# EUROPEAN PATENT APPLICATION

(11) **EP 4 169 616 A1**
(43) Date of publication of application: **26.04.2023**
(21) Application number: 21825210.4
(22) Date of filing: 19.06.2021
(51) Int. Cl.: B01L 3/00, B01L 3/14, G01N 33/52, G01N 33/558

(54) **BIOCHEMICAL TEST PAPER TUBE**

(30) Priority: 19.06.2020 CN 202010568656; 19.06.2020 CN 202021152064 U; 25.01.2021 CN 202110098826; 25.01.2021 CN 202120200290 U
(71) Applicant: Quicking Biotech Co., Ltd., Shanghai 201314 (CN); Amazing Biotech (Shanghai) Co., Ltd., Shanghai 201314 (CN); Shanghai Kinbio Tech Co., Ltd., Shanghai 201314 (CN)
(72) Inventor: LIU, Fang, Shanghai 201314 (CN); LI, Ziyue, Shanghai 201314 (CN); QIAN, Ruize, Shanghai 201314 (CN); WEI, Li, Shanghai 201314 (CN); ZHOU, Zhongren, Shanghai 201314 (CN)
(74) Representative: KIPA AB
(86) International application number: PCT/CN2021/101114
(87) International publication number: WO 2021/254515

(57) **Abstract**

A biochemical test strip tube comprises a tube portion and a cover portion. The tube portion comprises a solution chamber and a test strip fixing member which has a horizontal spacing between its outer edge and an inner wall of the tube portion. When the cover portion seals off the tube portion, a top of the solution chamber communicates with a top of the test strip fixing member, and a horizontal spacing is provided between an outer edge of the test strip fixing member and an inner wall of the cover portion. Due to the horizontal spacing between the test paper fixing member and the inner wall of the tube portion, the biochemical test strip does not contact or form a wall against the inner wall of the tube portion to avoid affecting the detection effect when the biochemical test strip is provided inside the test strip fixing member.

## Description

### CROSS REFERENCE TO RELATED APPLICATION(S)

This patent application claims the benefit and priority of Chinese Patent Application No. 202010568656.9 filed on June 19, 2020, Chinese Patent Application No. 202021152064.0 filed on June 19, 2020, Chinese Patent Application No. 202110098826.6 filed on January 25, 2021, and Chinese Patent Application No. 202120200290.X filed on January 25, 2021, the disclosure of which is incorporated by reference herein in its entirety as part of the present application.

### TECHNICAL FIELD

The present disclosure relates to the field of rapid detection device, and in particular, to a biochemical test strip tube.

### BACKGROUND

When performing in vitro diagnostics or in vitro testing, a combination of chromatography test strip and test tube is often used as a rapid diagnostic tool or rapid testing tool in order to obtain rapid qualitative results. In general, the samples used for diagnosis or testing are usually hazardous, such as toxic and infectious. The used tubes are disposed of as medical waste.

However, there are some defects of the test tubes in the related technology. For example, some test tubes have a separate design of tube portion and cover portion, which makes the cover portion easily fall off from the tube portion, causing material and solution inside the tube portion to flow outward which pollute the environment and endanger the life safety of the workers. In addition, some test tubes have a one-piece design of tube portion and cover portion, whose sealing and connection are not strong enough, which makes the cover portion easily separate from the tube portion under an action of external force, causing material and solution inside the tube portion to flow outward.

Therefore, there is no effective solution to the problem that the cover portion and the tube portion of the test tubes are easily separated and the substance inside the tube portion flows outward, which pollutes the environment and endangers the life of workers.

### SUMMARY

The present application overcomes the defects of the prior art and provides a biochemical test strip tube.

The present invention provides a biochemical test strip tube, comprising a tube portion and a cover portion which are internally inserted, wherein the tube portion comprises:
a solution chamber;
a test strip fixing member, wherein, when the cover portion seals off the tube portion, the test strip fixing member or a top of a sample receiving end of a biochemical test strip located within the test strip fixing member will extend a bottom end of an inner wall of the cover portion, with a vertical spacing of less than 2 mm from a top of the cover portion, and the test strip fixing member has a horizontal spacing between an outer edge of the test strip fixing member and an inner wall of a mouth of the tube portion, and the test strip fixing member or the top of the sample receiving end of the biochemical test strip located within the test strip fixing member will project at least 1 mm above the level of the mouth of the tube portion; and
the cover portion comprises:
   a cavity formed by an inner surface of the cover portion projecting outwardly.

Preferably, the test strip fixing member has a width of not more than 2.8 mm, and has a depth of not less than 28 mm.

Preferably, the horizontal spacing is greater than a wall thickness of an inner insertion port of the cover portion.

Preferably, the test strip fixing member is a biochemical test strip chamber, and a top of the biochemical test strip chamber communicates with the solution chamber.

Preferably, the test strip fixing member is a biochemical test strip container positioned by a snap-in side wall inside the solution chamber and an outer wall of the solution chamber, and the biochemical test strip container is provided with a connecting hole at a top of the biochemical test strip container which is connected to the sample solution receiving end of the biochemical test strip, or the biochemical test strip located inside the biochemical test strip container extends a specified length from the top of the biochemical test strip container.

Preferably, the test strip fixing member is a biochemical test strip container positioned by a snap-in side wall inside the solution chamber and an outer wall of the solution chamber, wherein, after a top end of the biochemical test strip container is folded, a fractured cross-section of the biochemical test strip container has a connecting hole connected to the sample solution receiving end of the biochemical test strip, or the biochemical test strip located inside the biochemical test strip container extends a specified length from the top of the biochemical test strip container.

Preferably, the cover portion further comprises:
a limit member provided on an outer wall of the cover portion; and
the biochemical test strip tube further comprises:
   an anti-opening member provided on the top of the tube portion and/or provided on the limit member of the cover portion.

Preferably, the cavity has a longitudinal cross-section in the shape of trapezoid, circle, or triangle.

Preferably, the anti-opening member comprises:
a countersink hole provided on a top of the mouth of the tube portion.

Preferably, the anti-opening member comprises:
a chamfered member provided on an outer wall of the limit member, when the cover portion seals off the tube portion, a width of a cross section of an upper end of the chamfered member is less than a width of a cross section of a lower end of the chamfered member

Preferably, the anti-opening member further comprises:
an opening member provided on the countersink hole, wherein, a first end of the opening member is located on an inner wall of the countersink hole, and a second end of the opening member is located on an outer wall of the countersink hole or between the outer wall of the countersink hole and the inner wall of the countersink hole.

Preferably, the cover portion further comprises:
a sealing member provided on an outer wall of the cover portion;
a first fitting member provided on the outer wall of the cover portion and located above the sealing member; and
the tube portion further comprises:
   a second fitting member provided on an inner wall of the tube portion, and the second fitting member can be nested and connected to the first fitting member.

Preferably, the sealing member is a ring-shaped projection.

Preferably, the first fitting member is a ring-shaped projection, and the second fitting member is a ring-shaped recess.

Preferably, a number of the first fitting members are provided, and a number of the first fitting members are spaced along the axial direction of the cover portion.

Preferably, a number of the second fitting members are provided, and a number of the second fitting members are spaced along the axial direction of the tube portion.

Preferably, the biochemical test strip tube further comprises:
a connecting member connected to the tube portion and the cover portion respectively.

Preferably, the biochemical test strip tube further comprises:
a biochemical test strip provided inside the test strip fixing member, wherein, when the cover portion seals off the tube portion, the sample receiving end of the biochemical test strip is proximate to the cover portion, and a horizontal spacing is provided between the sample receiving end of the biochemical test strip and the inner wall of the cover portion.

Preferably, the biochemical test strip protrudes from the test strip fixing member.

Preferably, a length of the biochemical test strip protruding the test strip fixing member is 1-5 mm.

Preferably, the biochemical test strip is an immunochromatographic test strip.

Preferably, the immunochromatographic test strip comprises:
a substrate;
a chromatography membrane provided on an upper surface of the substrate;
an absorbent pad provided on a lower surface of the substrate; and
the chromatography membrane and the absorbent pad are in contact at a tail end of the substrate and perform solution transfer.

Preferably, a tail end of the chromatography membrane extends a specified distance beyond the tail end of the substrate, a tail end of the absorbent pad extends a specified distance beyond the tail end of the substrate, and the tail end of the chromatography membrane and the tail end of the absorbent pad are in contact and perform solution transfer.

Preferably, the chromatography membrane is also provided on the lower surface of the substrate, and the tail end of the chromatography membrane and the tail end of the absorbent pad are in contact on the lower surface of the substrate and perform solution transfer.

Preferably, the absorbent pad is also provided on the upper surface of the substrate, and the tail end of the chromatography membrane and the tail end of the absorbent pad are in contact on the upper surface of the substrate and perform solution transfer.

Preferably, the immunochromatographic test strip further comprises:
a binding pad provided on the substrate, wherein a tail end of the binding pad is in contact with a front end of the chromatography membrane, and the binding pad delivers a solution to the chromatography membrane;
a guiding membrane provided on the substrate, wherein a tail end of the guiding membrane is in contact with a front end of the binding pad, and the guiding membrane delivers a solution to the binding pad; and
wherein, the solution chromatography rate of the guiding membrane is less than the solution chromatography rate of the binding pad.

Preferably, the guiding membrane has a thickness less than 0.15 mm.

Preferably, the immunochromatographic test strip further comprises:
a sample pad provided on the substrate, wherein, a tail end of the sample pad is in contact with a front end of the guiding membrane, and the sample pad delivers a solution to the guiding membrane.

Preferably, the immunochromatographic test strip further comprises:
a sample pad provided on the substrate, wherein, a tail end of the guiding membrane is in contact with a front end of the sample pad, and the guiding membrane delivers a solution to the sample pad.

Preferably, the immunochromatographic test strip further comprises:
a sample limit membrane provided on the substrate, wherein, a tail end of the sample limit membrane is in contact with a front end of the guiding membrane, and the sample limit membrane delivers a solution to the guiding membrane.

Preferably, the sample limit membrane has an aspiration saturation volume of 2~20 µl.

Preferably, the immunochromatographic test strip further comprises:
a filter pad provided on the substrate, wherein, a tail end of the guiding membrane is in contact with a front end of the filter pad, and the guiding membrane delivers a solution to the filter pad.

Preferably, the immunochromatographic test strip further comprises:
a water retaining pad provided near the front end of the guiding membrane.

Preferably, the water retaining pad fills a gap between the immunochromatographic test strip and the test strip fixing member.

Preferably, the immunochromatographic test strip further comprises:
a transparent protective film at least covering the chromatography membrane and the binding pad.

Preferably, the chromatography membrane comprises a detection line and a quality control line set sequentially.

Preferably, a lower surface of the tail end of the absorbent pad is in contact with an upper surface of the tail end of the chromatography membrane; or
an upper surface of the tail end of the absorbent pad is in contact with a lower surface of the tail end of the chromatography membrane.

Preferably, a lower surface of the tail end of the binding pad is in contact with an upper surface of the front end of the chromatography membrane; or
an upper surface of the tail end of the binding pad is in contact with a lower surface of the front end of the chromatography membrane.

Preferably, a lower surface of the tail end of the guiding membrane is in contact with an upper surface of the front end of the binding pad; or
an upper surface of the tail end of the guiding membrane is in contact with a lower surface of the front end of the binding pad.

Preferably, a lower surface of the tail end of the guiding membrane is in contact with an upper surface of the front end of the filter pad; or
an upper surface of the tail end of the guiding membrane is in contact with a lower surface of the front end of the filter pad.

Preferably, a lower surface of the tail end of the filter pad is in contact with an upper surface of the front end of the binding pad; or
an upper surface of the tail end of the filter pad is in contact with a lower surface of the front end of the binding pad.

Preferably, a lower surface of the tail end of the sample pad is in contact with an upper surface of the front end of the guiding membrane; or
an upper surface of the tail end of the sample pad is in contact with a lower surface of the front end of the guiding membrane.

Preferably, a lower surface of the tail end of the sample pad is in contact with an upper surface of the front end of the binding pad; or
an upper surface of the tail end of the sample pad is in contact with a lower surface of the front end of the binding pad.

Preferably, a lower surface of the tail end of the guiding membrane is in contact with an upper surface of the front end of the sample pad; or
an upper surface of the tail end of the guiding membrane is in contact with a lower surface of the front end of the sample pad.

Preferably, a lower surface of the tail end of the sample limit membrane is in contact with an upper surface of the front end of the guiding membrane; or
an upper surface of the tail end of the sample limit membrane is in contact with a lower surface of the front end of the guiding membrane.

Preferably, in the case where the tail end of the absorbent pad is located on the upper surface of the substrate and the front end of the absorbent pad is located on the lower surface of the substrate:
a length of the absorbent pad located on the upper surface of the substrate is greater than a length of the absorbent pad located on the lower surface of the substrate; or
a length of the absorbent pad located on the upper surface of the substrate is equal to a length of the absorbent pad located on the lower surface of the substrate; or
a length of the absorbent pad located on the upper surface of the substrate is less than a length of the absorbent pad located on the lower surface of the substrate.

Preferably, in the case where the front end of the chromatography membrane is located on the upper surface of the substrate and the tail end of the chromatography membrane is located on the lower surface of the substrate:
a length of the chromatography membrane located on the upper surface of the substrate is greater than a length of the chromatography membrane located on the lower surface of the substrate; or
a length of the chromatography membrane located on the upper surface of the substrate is equal to a length of the chromatography membrane located on the lower surface of the substrate; or
a length of the chromatography membrane located on the upper surface of the substrate is less than a length of the chromatography membrane located on the lower surface of the substrate.

Preferably, both the detection line and the quality control line are located on the upper surface of the substrate; or
the detection line is located on the upper surface of the substrate and the quality control line is located on the lower surface of the substrate; or
both the detection line and the quality control line are located on the lower surface of the substrate.

Preferably, the biochemical test strip tube further comprises:
a transparent hollow tube provided inside the test strip fixing member, and the transparent hollow tube is provided with the biochemical test strip.

Preferably, one end of the transparent hollow tube is a closed end.

Preferably, the test strip fixing member is a test strip cassette which comprises:
a bottom card which comprises:
a biochemical test strip slot provided on a first end surface of the bottom card for placing the biochemical test strip;
a first sealing surface provided around the biochemical test strip slot; and
a first sealing member bonded to the first sealing surface, which makes the biochemical test strip slot form a sealed space.

Preferably, the bottom card further comprises:
a sample solution addition hole provided on a second end surface of the bottom card, and connected to the biochemical test strip slot;
a second sealing surface provided around the sample solution addition hole; and
the test strip cassette further comprises:
   a second sealing member bonded to the second sealing surface, which makes the sample solution addition hole form a sealed space.

Preferably, the bottom card further comprises:
a first creasing member provided on the second end surface of the bottom card and provided close to the sample solution addition hole; and
wherein, after folding the bottom card along the first creasing member, the sample solution receiving end of the biochemical test strip provided inside the biochemical test strip slot is expose to a folded section of the first creasing member.

Preferably, after bending the bottom card along the first creasing member, the sample solution receiving end of the biochemical test strip provided inside the biochemical test strip slot extends for a length greater than 0.5 mm outside of the biochemical test strip slot.

Preferably, the first creasing member comprises:
a first creasing element provided on the second end surface of the bottom card and provided close to the sample solution addition hole; and
a second creasing element provided symmetrically at both ends of the first creasing element and connected to the first creasing element.

Preferably, the biochemical test strip provided inside the biochemical test strip slot is provided with a cavity at an outer end of an intersection of the first creasing member and the biochemical test strip, and a ratio of a height of the cavity to a length of the outer end of the biochemical test strip is greater than 70%.

Preferably, the bottom card further comprises:
a second creasing member provided on the first end surface of the bottom card, located inside the biochemical test strip slut corresponding to the first creasing member.

Preferably, the bottom card is made of transparent material; and/or
the first sealing member is a first sealing film, and the first sealing film comprises an aluminum foil film; and/or
the second sealing member is a second sealing film, and the second sealing film comprises an aluminum foil film.

Preferably, the bottom card further comprises:
a guide member provided on both sides of the bottom card and extended from one side of the first creasing member to the other side of the first creasing member;
the test strip cassette further comprises:
   a sliding member slidably connected to the guide member; and
   wherein, after bending the bottom card along the first creasing member, the bottom card is divided into a first part and a second part, both the first part and the second part are in a bent state, and the slide member is initially located in the first part; after the second part has been reset, both the first part and the second part are in a horizontal state, one end of the sliding member is located in the first part, the other end of the sliding member is located in the second part, and the sliding member seals the first creasing member.

Preferably, the bottom card further comprises:
an anti-releasing member provided on both ends of the guide member for limiting the sliding member.

The present application has the following advantages over the prior art: due to the horizontal spacing between the test paper fixing member and the inner wall of the tube portion, the biochemical test strip does not contact or form a wall against the inner wall of the tube portion to avoid affecting the detection effect when the biochemical test strip is provided inside the test strip fixing member.

### BRIEF DESCRIPTION OF THE DRAWINGS

FIG. 1 is a schematic diagram of a biochemical test strip tube according to embodiment 1 of the present invention.
FIG. 2 is a cross-sectional view of a closed state of the biochemical test strip tube according to embodiment 1 of the present invention.
FIG. 3 is a top view of an unclosed state of the biochemical test strip tube according to embodiment 1 of the present invention.
FIG. 4 is a cross-sectional view of the unclosed state of the biochemical test strip tube according to embodiment 1 of the present invention.
FIG. 5 is a cross-sectional view of a closed state of the biochemical test strip tube according to embodiment 2 of the present invention.
FIG. 6 is a cross-sectional view of the unclosed state of the biochemical test strip tube according to embodiment 3 of the present invention.
FIG. 7 is a cross-sectional view of the closed state of the biochemical test strip tube according to embodiment 4 of the present invention.
FIG. 8 is a top view of the unclosed state of the biochemical test strip tube according to embodiment 4 of the present invention.
FIG. 9 is a cross-sectional view of the unclosed state of the biochemical test strip tube according to embodiment 4 of the present invention.
FIG. 10 is a schematic diagram of a biochemical test strip tube according to embodiment 5 of the present invention.
FIG. 11 is a cross-sectional view of the closed state of the biochemical test strip tube according to embodiment 5 of the present invention.
FIG. 12 is a cross-sectional view of the unclosed state of the biochemical test strip tube according to embodiment 5 of the present invention.
FIG. 13 is a schematic diagram of a biochemical test strip tube according to embodiment 6 of the present invention.
FIG. 14 is a cross-sectional view of the closed state of the biochemical test strip tube according to embodiment 6 of the present invention.
FIG. 15 is a cross-sectional view of the unclosed state of the biochemical test strip tube according to embodiment 6 of the present invention.
FIG. 16 is a schematic diagram of a biochemical test strip tube according to embodiment 7 of the present invention.
FIG. 17 is a cross-sectional view of the closed state of the biochemical test strip tube according to embodiment 7 of the present invention.
FIG. 18 is a cross-sectional view of the closed state of the biochemical test strip tube according to embodiment 8 of the present invention.
FIG. 19 is a cross-sectional view of the unclosed state of the biochemical test strip tube according to embodiment 8 of the present invention.
FIG. 20 is a schematic diagram of an immunochromatographic test strip according to embodiment 9 of the present invention.
FIG. 21 is a cross-sectional view of the immunochromatographic test strip according to embodiment 9 of the present invention.
FIG. 22 is a cross-sectional view of the biochemical test strip tube according to embodiment 9 of the present invention (a biochemical test strip is provided inside the biochemical test strip tube).
FIG. 23 is a schematic diagram of the immunochromatographic test strip according to embodiment 10 of the present invention.
FIG. 24 is a cross-sectional view of the immunochromatographic test strip according to embodiment 10 of the present invention.
FIG. 25 is a schematic diagram of the immunochromatographic test strip according to embodiment 11 of the present invention.
FIG. 26 is a cross-sectional view of the immunochromatographic test strip according to embodiment 11 of the present invention.
FIG. 27 is a schematic diagram of the immunochromatographic test strip according to embodiment 12 of the present invention.
FIG. 28 is a cross-sectional view of the immunochromatographic test strip according to embodiment 12 of the present invention.
FIG. 29 is a cross-sectional view of the immunochromatographic test strip according to embodiment 13 of the present invention.
FIG. 30 is a cross-sectional view of a transparent hollow tube according to embodiment 14 of the present invention.
FIG. 31 is a cross-sectional view of the transparent hollow tube according to embodiment 14 of the present invention.
FIG. 32 is a cross-sectional view of the biochemical test strip tube according to embodiment 14 of the present invention (a biochemical test strip with a transparent hollow tube is provided inside the biochemical test strip tube).
FIG. 33 is a schematic diagram of a test strip cassette according to embodiment 15 of the present invention.
FIG. 34 is a schematic diagram of the test strip cassette according to embodiment 15 of the present invention.
FIG. 35 is a schematic diagram of a bottom card according to embodiment 15 of the present invention.
FIG. 36 is a schematic diagram of the bottom card assembled with a biochemical test strip according to embodiment 15 of the present invention.
FIG. 37 is a schematic diagram of a test strip cassette according to embodiment 16 of the present invention.
FIG. 38 is a schematic diagram of the test strip cassette according to embodiment 16 of the present invention.
FIG. 39 is a schematic diagram of a bottom card according to embodiment 16 of the present invention.
FIG. 40 is a schematic diagram of the bottom card according to embodiment 16 of the present invention.
FIG. 41 is a schematic diagram of the bottom card assembled with a biochemical test strip according to embodiment 16 of the present invention.
FIG. 42 is a schematic diagram of the bottom card assembled with a biochemical test strip according to embodiment 16 of the present invention.
FIG. 43 is a schematic diagram of a test strip cassette according to embodiment 17 of the present invention.
FIG. 44 is a schematic diagram of the test strip cassette according to embodiment 17 of the present invention.
FIG. 45 is a schematic diagram of a bottom card according to embodiment 17 of the present invention.
FIG. 46 is a schematic diagram of the bottom card according to embodiment 17 of the present invention.
FIG. 47 is a schematic diagram of the bottom card assembled with a biochemical test strip according to embodiment 17 of the present invention.
FIG. 48 is a schematic diagram of the bottom card assembled with a biochemical test strip according to embodiment 17 of the present invention.
FIG. 49 is a schematic diagram of a test strip cassette according to embodiment 18 of the present invention.
FIG. 50 is a schematic diagram of the test strip cassette according to embodiment 18 of the present invention.
FIG. 51 is a schematic diagram of a bottom card according to embodiment 18 of the present invention.
FIG. 52 is a schematic diagram of the bottom card according to embodiment 18 of the present invention.
FIG. 53 is a schematic diagram of the bottom card assembled with a biochemical test strip according to embodiment 18 of the present invention.
FIG. 54 is a schematic diagram of the bottom card assembled with a biochemical test strip according to embodiment 18 of the present invention.
FIG. 55 is a schematic diagram of a test strip cassette according to embodiment 19 of the present invention.
FIG. 56 is a schematic diagram of a bottom card according to embodiment 18 of the present invention.
FIG. 57 is a schematic diagram of the bottom card assembled with a biochemical test strip according to embodiment 19 of the present invention.
FIG. 58 is a schematic diagram of the bottom card assembled with a biochemical test strip according to embodiment 19 of the present invention.
FIG. 59 is a schematic diagram of a bottom card according to embodiment 20 of the present invention.
FIG. 60 is a schematic diagram of the bottom card according to embodiment 20 of the present invention.
FIG. 61 is a cross-sectional view of the bottom card according to embodiment 20 of the present invention.

Reference numerals: biochemical test strip tube: 100; tube portion: 110; solution chamber: 111; test strip fixing member: 112; snap-in side wall: 113; second fitting member: 114; cover portion: 120; limit member: 121; cavity: 122; sealing member: 123; first fitting member: 124; connecting member: 130; anti-opening member: 140; countersink hole: 141; chamfered member: 142; opening member: 143;
immunochromatographic test strip: 200; substrate: 210; chromatography membrane: 220; detection line: 221; quality control line: 222; absorbent pad: 230; binding pad: 240; guiding membrane: 250; sample pad: 260; sample limit membrane: 270; transparent protective film:280
transparent hollow tube: 300;
test strip cassette: 400; bottom card: 410; biochemical test strip slot: 411; first sealing surface: 412; sample solution addition hole: 413; second sealing surface: 414; first creasing member: 415; first creasing element: 4151; second creasing element: 4152; second creasing member: 416; guide member: 417; anti-releasing member: 418; first sealing member: 420; second sealing member: 430; sliding member: 440.

### DETAILED DESCRIPTION

The technical solutions in the examples of the present disclosure are clearly and completely described below with reference to the accompanying drawings in the examples of the present disclosure. Apparently, the described examples are merely a part rather than all of the examples of the present disclosure. All other examples obtained by a person of ordinary skill in the art based on the examples of the present disclosure without creative efforts shall fall within the protection scope of the present disclosure.

It should be noted that the examples in the present disclosure or features in the examples may be combined in a non-conflicting manner.

The present disclosure is further described below with reference to the accompanying drawings and specific examples, but the present disclosure is not limited thereto.

### Embodiment 1

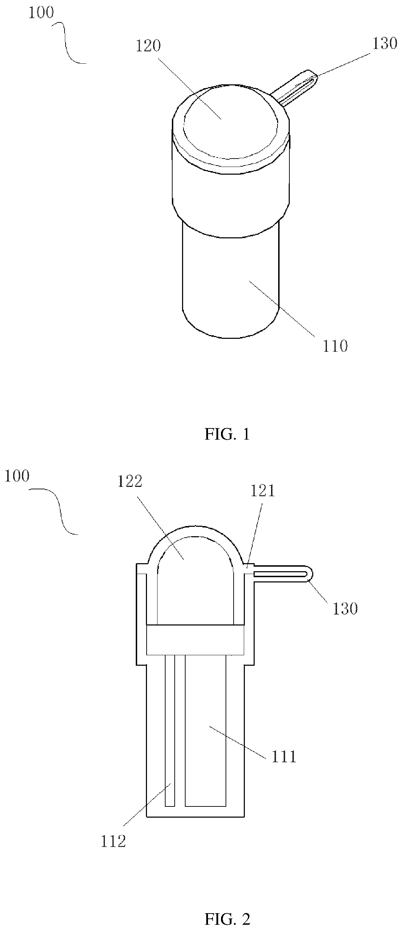

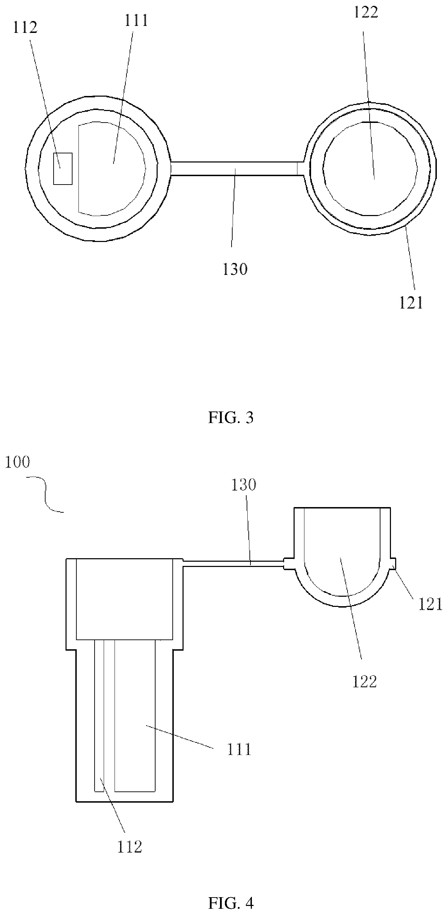

According to an exemplary example of the present invention, as shown in FIG. 1 to FIG. 4, a biochemical test strip tube 100 comprises a tube portion 110, a cover portion 120 and a connecting member 130, wherein, the tube portion 110 and the cover portion 120 are connected by the connecting member 130, and the tube portion 110 and the cover portion 120 are internally inserted.

The tube portion 110 comprises a solution chamber 111 and a test strip fixing member 112. An axial direction of the solution chamber 111 is co-linear or parallel to an axial direction of the tube portion 110, and the solution chamber 111 is used to store a solution, such as a reaction solution. An axial direction of the test strip fixing member 112 is parallel to the axial direction of the solution chamber 111, and the test strip fixing member 112 is used to place a biochemical test strip.

Preferably, the test strip fixing member 112 has a horizontal spacing, at least 0.5 mm, between an outer edge of the test strip fixing member 112 and an inner wall of the tube portion 110 for preventing the biochemical test strip placed in the test strip fixing member 112 from contacting or forming a wall against the inner wall of the tube portion 110.

Preferably, the test strip fixing member 112 has a width (in a direction of the diameter of the tube portion 110) of not more than 2.8 mm, and has a depth of not less than 28 mm.

Preferably, when the cover portion 120 seals off the tube portion 110, a top of the solution chamber 111 communicates with a top of the test strip fixing member 112.

Preferably, when the cover portion 120 seals off the tube portion 110, the test strip fixing member 112 has a horizontal spacing, at least 0.5 mm, between an inner edge of the test strip fixing member 112 and an inner wall of the cover portion 120 for preventing the biochemical test strip placed in the test strip fixing member 112 from contacting or forming a wall against the inner wall of the cover portion 120.

Preferably, when the cover portion 120 seals off the tube portion 110, the test strip fixing member 112 has a horizontal spacing, at least 0.5 mm, between an outer edge of the test strip fixing member 112 and an inner wall of the cover portion 120 for preventing the biochemical test strip placed in the test strip fixing member 112 from contacting or forming a wall against the inner wall of the cover portion 120.

Preferably, when the cover portion 120 seals off the tube portion 110, the test strip fixing member 112 has a vertical spacing, less than 2 mm, between a top wall of the test strip fixing member 112 and a bottom wall of the cover portion 120.

Preferably, the solution chamber 111 is a regular shaped chamber (e.g., circular chamber, rectangular chamber, trapezoidal chamber) or an irregular shaped chamber. The test strip fixing member 112 is a regular shaped chamber (e.g., circular chamber, rectangular chamber, trapezoidal chamber) or an irregular shaped chamber.

Preferably, the test strip fixing member 112 is a biochemical test strip chamber, and a top of the test strip fixing member 112 communicates with the top of the solution chamber 111.

In one of the embodiments, the number of the solution chamber 111 may be a plurality. The axes of the plurality of the solution chambers 111 are parallel. At least one solution chamber 111 is used to store a biochemical reaction reagent or a biochemical reaction solution, and at least one solution chamber 111 is used to store a functional reaction reagent. Wherein, the functional reaction reagent includes, but is not limited to, a degrading enzyme, a lysis solution, a probe, a buffer, a diluent, and the like, or reagents for subsequent reactions after the biochemical reaction is completed.

In one of the embodiments, the number of the test strip fixing member 112 may be a plurality. The axes of the plurality of the test strip fixing members 112 are parallel. Each test strip fixing member 112 is used to place a biochemical test strip for allowing simultaneous testing of different indicators of the same sample.

Preferably, the plurality of the test strip fixing member 112 are provided around the axial direction of the tube portion 110.

The cover portion 120 comprises a limit member 121 and a cavity 122. The limit member 121 is provided on the outer wall of the cover portion 120, and the cavity 122 is formed by the outward projection of the inner surface of the cover 120.

Preferably, the limit member 121 is a limit ring.

Preferably, a longitudinal section of the cavity 122 is trapezoidal, circular or triangular.

The connecting member 130 is connected to the tube portion 110 and the cover portion 120 respectively, so that the tube portion 110 and the cover portion 120 form a one-piece structure.

Preferably, the connecting member 130 is a connecting strip, one end of which is connected to the tube portion 110 and the other end of which is connected to the cover portion 120.

In this embodiment, due to the specifically designed test strip fixing member of the biochemical test strip tube, the biochemical test strip provided inside the test strip fixing member does not contact either the inner wall of the tube portion or the interior of the cover portion for avoiding the biochemical test strip from sticking to the wall.

### Embodiment 2

This embodiment is a variation of embodiment 1. The difference between this embodiment and embodiment 1 is that the fitting method of the tube portion 110 and the cover portion 120 is different.

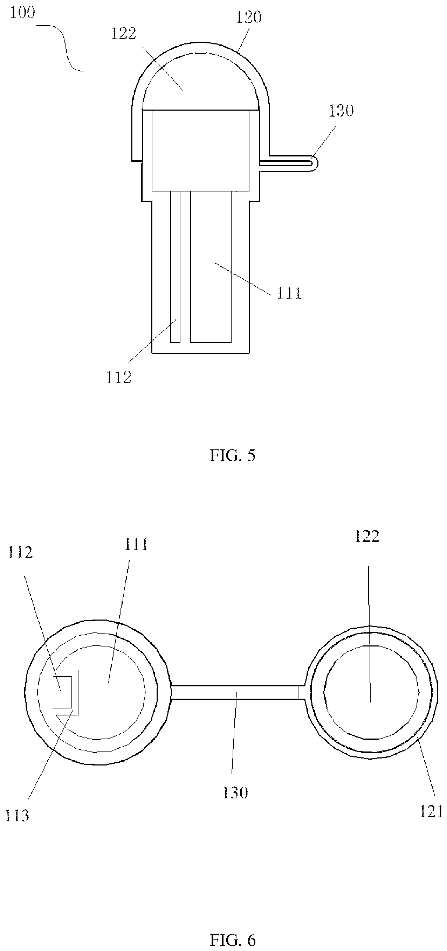

As shown in FIG. 5, the tube portion 110 and the cover portion 120 are externally inserted. Specifically, the maximum inner diameter of the cover portion 120 is equal to the outer diameter of the tube portion 110, which means the cover portion 120 is snapped to the outside of the mouth of the tube portion 110.

### Embodiment 3

This embodiment is a variation of embodiment 1. The difference between this embodiment and embodiment 1 is that the test strip fixing member 112 is different.

As shown in FIG. 6, the test strip fixing member 112 is a biochemical test strip container positioned by a snap-in side wall 113 inside the solution chamber 111 and an outer wall of the solution chamber 111, and the biochemical test strip container is provided with a connecting hole at a top of the biochemical test strip container which is connected to the sample solution receiving end of the biochemical test strip, or the biochemical test strip located inside the biochemical test strip container extends a specified length from the top of the biochemical test strip container.

### Embodiment 4

This embodiment is a variation of embodiment 3. The difference between this embodiment and embodiment 3 is that the test strip fixing member 112 is different.

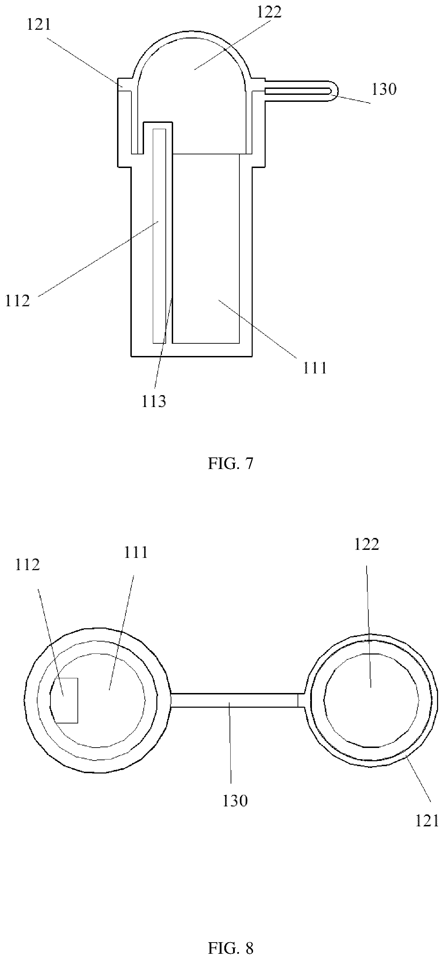

As shown in FIG. 7 to FIG. 9, the test strip fixing member 112is a biochemical test strip container positioned by a snap-in side wall 113 inside the solution chamber 111 and an outer wall of the solution chamber 111, wherein, after a top end of the biochemical test strip container is folded, a fractured cross-section of the biochemical test strip container has a connecting hole connected to the sample solution receiving end of the biochemical test strip, or the biochemical test strip located inside the biochemical test strip container extends a specified length from the top of the biochemical test strip container.

### Embodiment 5

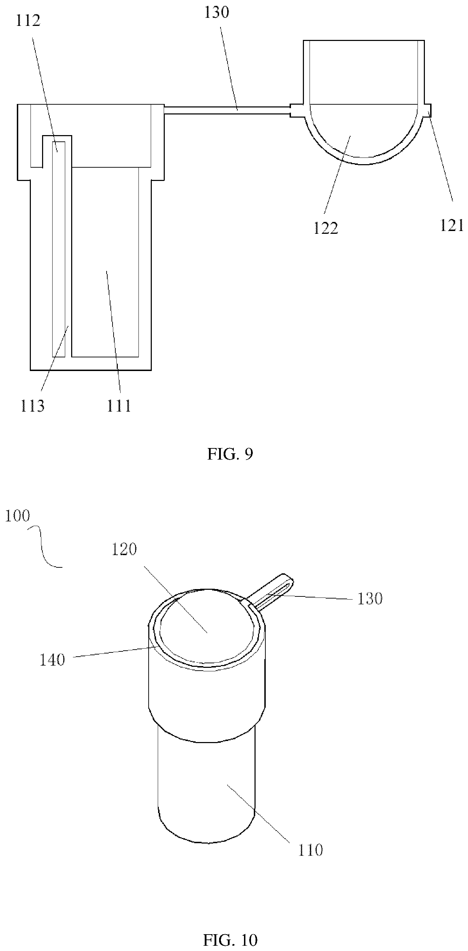

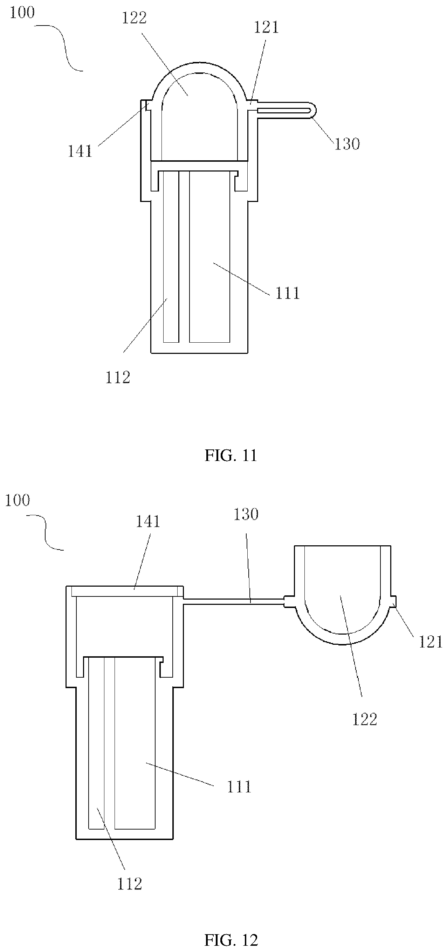

As shown in FIG. 10 to FIG. 12, a biochemical test strip tube 100 comprises a tube portion 110, a cover portion 120, a connecting member 130 and an anti-opening member 140, wherein, the tube portion 110 and the cover portion 120 are connected by the connecting member 130, and the anti-opening member 140 is provided on the top of the tube portion 110. Wherein, when the cover portion 120 seals off the tube portion 110, the cover portion 120 cannot be removed from the tube portion 110 by the action of the anti-opening member 140.

Preferably, the structure and the connection of the tube portion 110, the cover portion 120 and the connecting member 130 are basically the same as in embodiment 1, which will not be repeated here.

The anti-opening member 140 comprises a countersink hole 141. The countersink hole 141 is provided on the top of the tube portion 110, which is capable of cooperating with the limit member 121 of the cover portion 120. Wherein, when the cover portion 120 seals off the tube portion 110, the limit member 121 is provided in the countersink hole 141, and an upper surface of the limit member 121 is at the same level with an upper surface of the countersink hole 141.

The connecting member 130 is connected to the tube portion 110 and the cover portion 120 respectively, so that the tube portion 110 and the cover portion 120 form a one-piece structure.

Preferably, the connecting member 130 is a connecting strip, one end of which is connected to the tube portion 110 and the other end of which is connected to the cover portion 120.

This embodiment is used as follows: placing a biochemical test strip into the test strip fixing member 112 with the sample solution receiving end of the biochemical test strip facing upward; putting a reaction solution into the solution chamber 111 and putting the sample, such as a throat swab, into the solution chamber 111; sealing off the tube portion 110 by the cover portion 120 and making the limit member 121 of the cover portion 120 submerged in the countersink hole 141; after the reaction in the solution chamber 111 is complete, titling the biochemical test strip tube 100, so that the reaction solution contacts to the biochemical test strip and undergoes biochemical reaction on the biochemical test strip, and the test result will be obtained by observing the biochemical test strip; and after observing the test result, the biochemical test strip tube can be directly disposed of as medical waste without worrying about the leakage of substances inside the biochemical test strip tube 100.

In this embodiment, due to the biochemical test strip tube with the anti-opening member, after the cover portion seals off the tube portion, it is difficult to separate the cover portion from the tube portion, which prevents the leakage of substances inside the biochemical test strip tube, and prevents pollution of the environment and health hazards.

### Embodiment 6

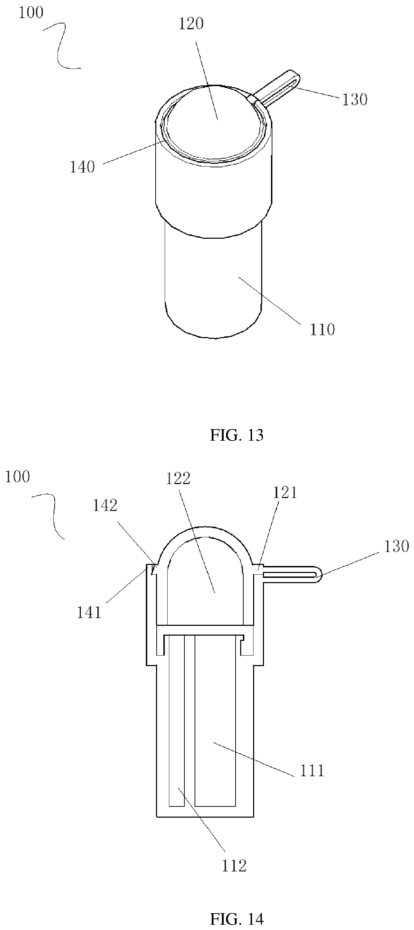

As shown in FIG. 13 to FIG. 15, a biochemical test strip tube 100 comprises a tube portion 110, a cover portion 120, a connecting member 130 and an anti-opening member 140, wherein, the tube portion 110 and the cover portion 120 are connected by the connecting member 130, and the anti-opening member 140 is provided on the top of the tube portion 110 and the outer wall of the cover portion 120. Wherein, when the cover portion 120 seals off the tube portion 110, the cover portion 120 cannot be removed from the tube portion 110 by the action of the anti-opening member 140.

Preferably, the structure and the connection of the tube portion 110, the cover portion 120 and the connecting member 130 are basically the same as in embodiment 1, which will not be repeated here.

The anti-opening member 140 comprises a countersink hole 141 and a chamfered member 142. The countersink hole 141 is provided on the top of the tube portion 110, and the chamfered member 142 is provided on the outer wall of the limit member 121.

Preferably, the chamfered member 142 and the limit member 121 are of an integrated design.

Preferably, when the cover portion 120 seals off the tube portion 110, a width of the cross-section of a first end (an upper end) of the chamfered member 142 is less than a width of the cross-section of a second end (a lower end) of the chamfered member 142.

Preferably, when the cover portion 120 seals off the tube portion 110, the limit member 121 together with the chamfered member 142 is provided in the countersink hole 141. An upper surface of the limit member 121, an upper surface of the chamfered member 142 and an upper surface of the countersink hole 141 are at the same level.

The use of this embodiment is basically the same as that of embodiment 5, which will not be repeated here.

In this embodiment, when the cover portion seals off the tube portion, it is difficult to separate the cover portion from the tube portion under the dual action of the countersink hole and the chamfered member. Since the chamfered member cannot provide a force point, even if an external force is forced on the countersink hole and the chamfered member, it is difficult to separate the cover portion from the tube portion.

### Embodiment 7

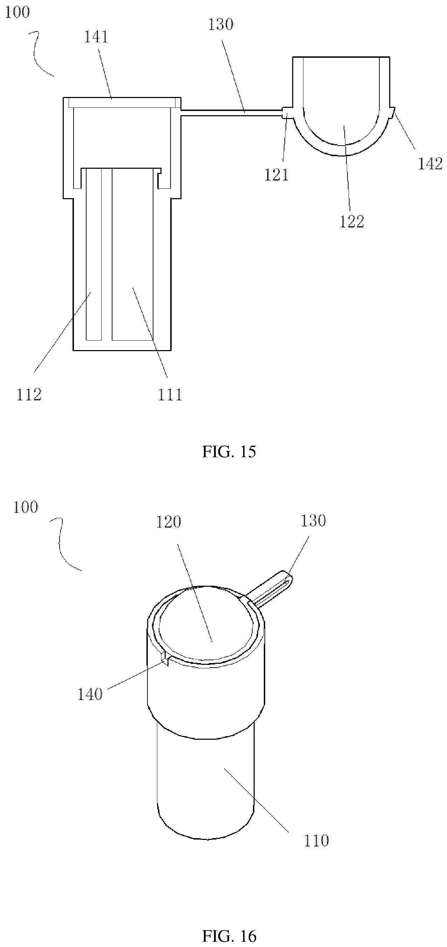

As shown in FIG. 16 to FIG. 17, a biochemical test strip tube 100 comprises a tube portion 110, a cover portion 120, a connecting member 130 and an anti-opening member 140, wherein, the tube portion 110 and the cover portion 120 are connected by the connecting member 130, and the anti-opening member 140 is provided on the top of the tube portion 110. Wherein, when the cover portion 120 seals off the tube portion 110, the cover portion 120 cannot be removed from the tube portion 110 by the action of the anti-opening member 140.

Preferably, the structure and the connection of the tube portion 110, the cover portion 120 and the connecting member 130 are basically the same as in embodiment 1, which will not be repeated here.

The anti-opening member 140 comprises a countersink hole 141 and an opening member 143.

Wherein, the countersink hole 141 is provided on the top of the tube portion 110, which is capable of cooperating with the limit member 121 of the cover portion 120. Wherein, when the cover portion 120 seals off the tube portion 110, the limit member 121 is provided in the countersink hole 141, and an upper surface of the limit member 121 is at the same level with an upper surface of the countersink hole 141.

Wherein, the opening member 143 is provided on an upper surface or the interior of the countersink hole 141 for separating the cover portion 120 from the tube portion 110 in a particular case.

In one of the embodiments, the opening member 143 is provided through the countersink hole 141. Specifically, a first end of the opening member 143 is disposed on an inner wall of the countersink hole 141, and a second end of the opening member 143 is disposed on an outer wall of the countersink hole 141. The cover portion 120 is cocked by passing through the opening member 143 with an auxiliary tool, such as a pry bar, to separate the cover portion 120 from the tube portion 110.

In one of the embodiments, the opening member 143 is provided on the upper surface of the countersink hole 141. Specifically, a first end of the opening member 143 is disposed on an inner wall of the countersink hole 141, and a second end of the opening member 143 is disposed between the inner wall and an outer wall of the countersink hole 141. The cover portion 120 is cocked by passing through the opening member 143 with an auxiliary tool, such as a pry piece, to separate the cover portion 120 from the tube portion 110.

The use of this embodiment is substantially the same as that of embodiment 5, with the difference that after the test result is known, the cover portion 120 can be separated from the tube portion 110 by the opening member 143 to recover the solution or substance inside the biochemical test strip tube for disposal.

### Embodiment 8

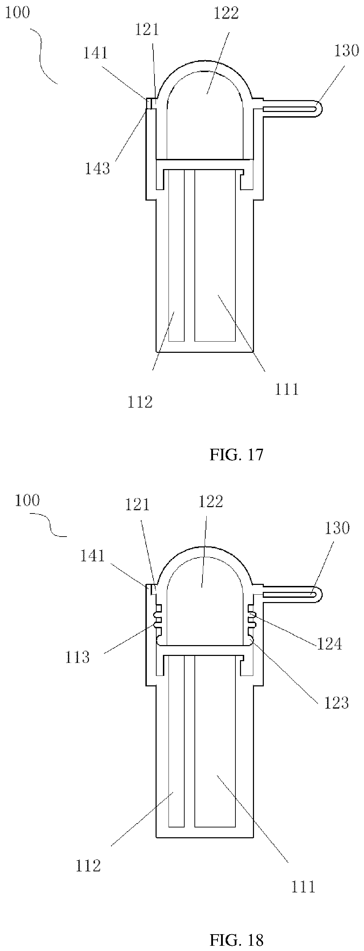

As shown in FIG. 18 to FIG. 19, a biochemical test strip tube 100 comprises a tube portion 110, a cover portion 120, a connecting member 130 and an anti-opening member 140, wherein, the tube portion 110 and the cover portion 120 are connected by the connecting member 130, and the anti-opening member 140 is provided on the top of the tube portion 110. Wherein, when the cover portion 120 seals off the tube portion 110, the cover portion 120 cannot be removed from the tube portion 110 by the action of the anti-opening member 140.

Preferably, the structure and the connection of the connecting member 130 and the anti-opening member 140 are basically the same as in embodiment 5 to embodiment 7, which will not be repeated here.

The tube portion 110 comprises a solution chamber 111, a test strip fixing member 112 and a second fitting member 114.

Preferably, the structure and the connection of the solution chamber 111 and the test strip fixing member 112 are basically the same as in embodiment 1, which will not be repeated here.

The second fitting member 114 is provided on an inner wall of the top of the tube portion 110, and the second fitting member 114 is spaced along the axial direction of the tube portion 110.

The cover portion 120 comprises a limit member 121, a cavity 122, a sealing member 123 and a first fitting member 124.

Preferably, the structure and the connection of the limit member 121 and the cavity 122 are basically the same as in embodiment 1, which will not be repeated here.

The sealing member 123 and the first fitting member 124 are spaced along the axial direction of the cover portion 120. When the cover portion 120 seals off the tube portion 110, the sealing member 123 is disposed on a lower side of the cover portion 120, and the first fitting member 124 is disposed on an upper side of the sealing member 123.

When the cover portion 120 seals off the tube portion 110, the sealing member 123 is sealed to the top of the tube portion 110, and the first fitting member 124 is nested and connected to the second fitting member 114 to provide a tight connection between the cover portion 120 and the tube portion 110.

In one of embodiments, the sealing member 123 is a ring-shaped projection. The outer diameter of the sealing member 123 is equal to the inner diameter of the top of the tube portion 110.

In one of embodiments, the first fitting member 124 is a ring-shaped projection, and the outer diameter of the first fitting member 124 is greater than the inner diameter of the top of the tube portion 110. The second fitting member 114 is a ring-shaped recess, and fits snugly into the first fitting member 124.

In this embodiment, the sealing member 123, the first fitting member 124 and the second fitting member 114 are used to further improve the tightness of the connection between the cover portion 120 and the tube portion 110 and greatly improve the resistance of the separating the cover portion 120 from the tube portion 11 , which effectively prevents the leakage of the solution inside the biochemical test strip tube 100, and prevents pollution of the environment and health hazards.

### Embodiment 9

This embodiment relates to the biochemical test strip placed in the biochemical test strip tube of embodiment 1 to embodiment 8.

In this embodiment, the biochemical test strip is illustrated as an immunochromatographic test strip.

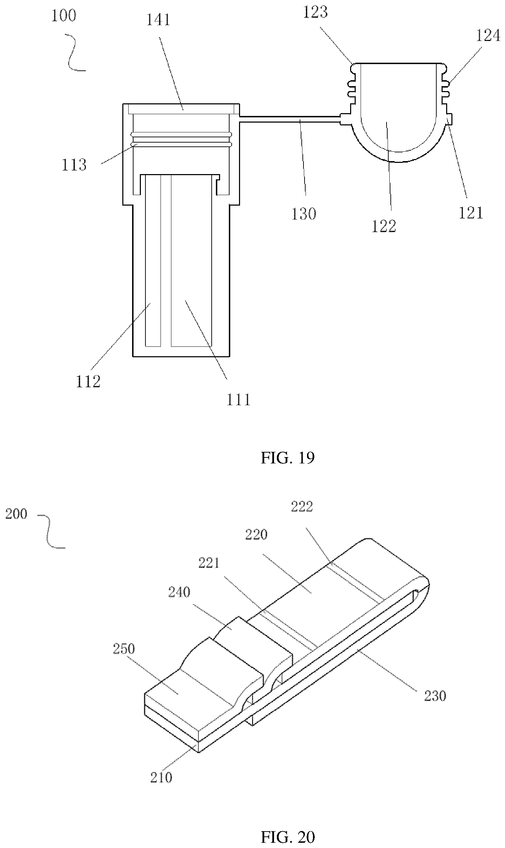

As shown in FIG. 20 to FIG. 22, the immunochromatographic test strip 200 comprises a substrate 210, a chromatography membrane 220, an absorbent pad 230, a binding pad 240 and a guiding membrane 250. Wherein, the guiding membrane 250, the binding pad 240, the chromatography membrane 220 and the absorbent pad 230 are provided sequentially on the substrate 210.

Preferably, the immunochromatographic test strip 200 protrudes from the test strip fixing member 112. Generally, a length of the biochemical test strip 200 protruding the test strip fixing member 112 is 1-5 mm. Preferably, the length is 2 mm or 3 mm.

Preferably, when the cover portion 120 seals off the tube portion 110, the sample receiving end of the immunochromatographic test strip 200 extends into the cavity 122 of the cover portion 120.

Furthermore, when the cover portion 120 seals off the tube portion 110, the immunochromatographic test strip 200 has a horizontal spacing between the sample receiving end of the immunochromatographic test strip 200 and an inner wall of the cover portion 120, at least 0.5 mm.

Preferably, the substrate 210 is made of plastic, such as self-adhesive plastic.

The guiding membrane 250 is provided on an upper surface of the substrate 210. That is, a lower surface of the guiding membrane 250 is connected to the upper surface of the substrate 210, such as by bonding. A tail end of the guiding membrane 250 is in contact with a front end of the binding pad 240, and the guiding membrane 250 delivers a solution to the bonding pad 240.

Wherein, an upper surface of the tail end of the guiding membrane 250 is in contact with a lower surface of the front end of the binding pad 240, or, a lower surface of the tail end of the guiding membrane 250 is in contact with an upper surface of the front end of the binding pad 240.

Preferably, the guiding membrane 250 is made of cellulose membrane.

Preferably, the guiding membrane 250 has a thickness less than 0.15 mm.

The binding pad 240 is provided on the upper surface of the substrate 210. That is, a lower surface of the binding pad 240 is connected to the upper surface of the substrate 210, such as by bonding. A tail end of the binding pad 240 is in contact with a front end of the chromatography membrane 220, and the binding pad 240 delivers a solution to the chromatography membrane 220. Specifically, an upper surface of the tail end of the binding pad 240 is in contact with a lower surface of the front end of the chromatography membrane 220, or, a lower surface of the tail end of the binding pad 240 is in contact with an upper surface of the front end of the chromatography membrane 220.

Preferably, the binding pad 240 is a colloidal gold pad.

Preferably, the binding pad 240 is made of glass fiber, polyester film, cellulose filter paper, non-woven fabric and so on.

Preferably, the solution chromatography rate of the guiding membrane 250 is less than the solution chromatography rate of the binding pad 240.

The chromatography membrane 220 is provided at least on the upper surface of the substrate 210. That is, a lower surface of the chromatography membrane 220 is connected to the upper surface of the substrate 210, such as by bonding. A tail end of the chromatography membrane 220 is in contact with a tail end of the absorbent pad 230, and the chromatography membrane 220 delivers a solution to the absorbent pad 230.

Preferably, the chromatography membrane 220 comprises a detection line 221 and a quality control line 222. Wherein, the detection line 221 and the quality control line 222 are provided sequentially along the front end to the tail end of the chromatography membrane 220.

Preferably, the chromatography membrane 220 is made of nitrocellulose membrane.

The absorbent pad 230 is provided at least on a lower surface of the substrate 210. That is, an upper surface of the absorbent pad 230 is connected to the lower surface of the substrate 210, such as by bonding. A tail end of the absorbent pad 230 is in contact with a tail end of the chromatography membrane 220.

Preferably, the absorbent pad 230 is an absorbent paper.

Preferably, the tail end of the chromatography membrane 220 and the tail end of the absorbent pad 230 are in contact at the tail end of the substrate 210, and the chromatography membrane 220 delivers a solution to the absorbent pad 230.

In a first example of this embodiment, the tail end of the chromatography membrane 220 extends a specified distance beyond the tail end of the substrate 210, the tail end of the absorbent pad 230 extends a specified distance beyond the tail end of the substrate 210, the tail end of the chromatography membrane 220 and the tail end of the absorbent pad 230 are in contact, and the chromatography membrane 220 delivers a solution to the absorbent pad 230. That is, the lower surface of the tail end of the chromatography membrane 220 and the upper surface of the tail end of the absorbent pad 230 are in contact and the solution is transferred. In this example, the chromatography membrane 220, the substrate 210 and the absorbent pad 230 form a sandwich structure.

In a second example of this embodiment, the chromatography membrane 220 is also provided on the lower surface of the substrate 210. That is, the tail end of the chromatography membrane 220 is bent downward at the tail end of the substrate 210. Specifically, the upper surface of the tail end of the chromatography membrane 220 and the upper surface of the tail end of the absorbent pad 230 are in contact and the solution is transferred, or the lower surface of the tail end of the chromatography membrane 220 and the lower surface of the tail end of the absorbent pad 230 are in contact and the solution is transferred.

In this second example, a length of the chromatography membrane 220 located on the upper surface of the substrate 210 is greater than a length of the chromatography membrane 220 located on the lower surface of the substrate 210; or, a length of the chromatography membrane 220 located on the upper surface of the substrate 210 is equal to a length of the chromatography membrane 220 located on the lower surface of the substrate 210; or, a length of the chromatography membrane 220 located on the upper surface of the substrate 210 is less than a length of the chromatography membrane 220 located on the lower surface of the substrate 210.

Preferably, the length of the chromatography membrane 220 located on the upper surface of the substrate 210 is greater than the length of the chromatography membrane 220 located on the lower surface of the substrate 210.

In a third example of this embodiment, the absorbent pad 230 is also provided on the upper surface of the substrate 210. That is, the tail end of the absorbent pad 230 is bent upward at the tail end of the substrate 210. Specifically, the upper surface of the tail end of the absorbent pad 230 and the upper surface of the tail end of the chromatography membrane 220 are in contact and the solution is transferred, or the lower surface of the tail end of the absorbent pad 230 and the lower surface of the tail end of the chromatography membrane 220 are in contact and the solution is transferred.

In this third example, a length of the absorbent pad 230 located on the upper surface of the substrate 210 is greater than a length of the absorbent pad 230 located on the lower surface of the substrate 210; or, a length of the absorbent pad 230 located on the upper surface of the substrate 210 is equal to a length of the absorbent pad 230 located on the lower surface of the substrate 210; or, a length of the absorbent pad 230 located on the upper surface of the substrate 210 is less than a length of the absorbent pad 230 located on the lower surface of the substrate 210.

Preferably, the length of the absorbent pad 230 located on the upper surface of the substrate 210 is less than the length of the absorbent pad 230 located on the lower surface of the substrate 210.

In addition, to ensure that the tail end of the chromatography membrane 220 and the tail end of the absorbent pad 230 are in contact, a film is provided on the tail end of the chromatography membrane 220 and the tail end of the absorbent pad 230. The film wraps around the chromatography membrane 220 and the absorbent pad 230 to keep the chromatography membrane 220 and the absorbent pad 230 in contact.

In this embodiment, the immunochromatographic test strip 200 has a length of 2 cm ~ 4 cm. Preferably, the length is 2.8 cm, 3.0 cm, 3.2 cm or 3.5 cm.

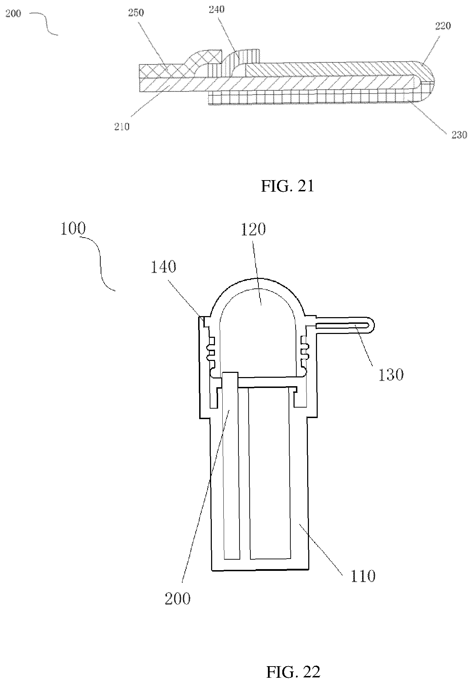

This embodiment is used as follows: as shown in FIG. 22, placing the immunochromatographic test strip 200 into the test strip fixing member 112 of the biochemical test strip tube 100 with the sample solution receiving end (that is the guiding membrane 250) of the immunochromatographic test strip 200 facing upward; putting a reaction solution into the solution chamber 111 and putting the sample into the solution chamber 111; after the reaction in the solution chamber 111 is complete, titling the biochemical test strip tube 100, so that the reaction solution contacts to the sample solution receiving end of the immunochromatographic test strip 200, and the reaction solution is rapidly adsorbed to the chromatography membrane 220 for color development by the action of absorbent pad 230.

### Embodiment 10

This embodiment relates to the biochemical test strip placed in the biochemical test strip tube of embodiment 1 to embodiment 8.

In this embodiment, the biochemical test strip is illustrated as an immunochromatographic test strip.

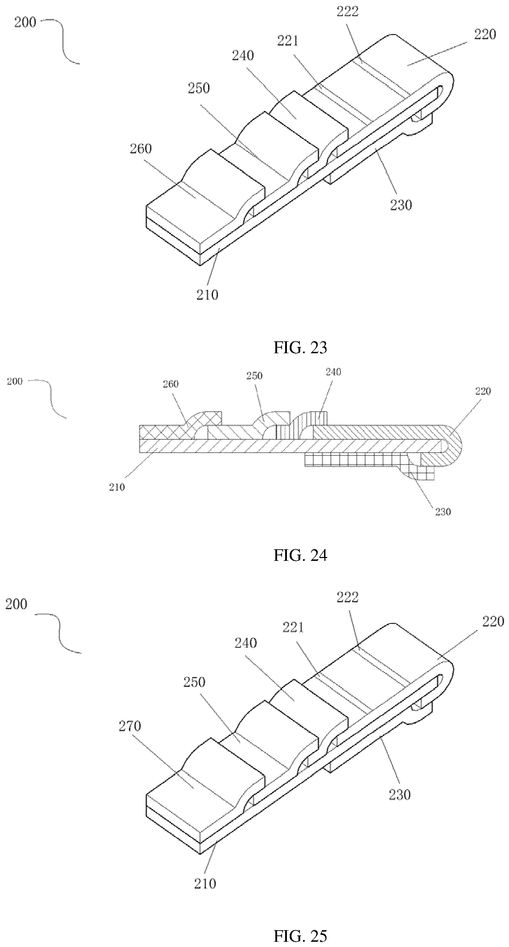

As shown in FIG. 23 to FIG. 24, the immunochromatographic test strip 200 comprises a substrate 210, a chromatography membrane 220, an absorbent pad 230, a binding pad 240, a guiding membrane 250 and a sample pad 260. Wherein, the sample pad 260, the guiding membrane 250, the binding pad 240, the chromatography membrane 220 and the absorbent pad 230 are provided sequentially on the substrate 210.

Preferably, the structure, the connection and the composition of the substrate 210, the chromatography membrane 220, the absorbent pad 230 and the binding pad 240 are basically the same as in embodiment 9, which will not be repeated here.

The sample pad 260 is provided on the upper surface of the substrate 210. That is, a lower surface of the sample pad 260 is connected to the upper surface of the substrate 210, such as by bonding.

Preferably, the sample pad 260 is made of glass fiber, polyester film, cellulose filter paper, non-woven fabric and so on.

In a first example of this embodiment, a tail end of the sample pad 260 is in contact with a front end of the guiding membrane 250, and the sample pad 260 delivers a solution to the guiding membrane 250. Specifically, an upper surface of the tail end of the sample pad 260 is in contact with a lower surface of the front end of the guiding membrane 250, or, a lower surface of the tail end of the sample pad 260 is in contact with an upper surface of the front end of the guiding membrane 250.

In a second example of this embodiment, a front end of the sample pad 260 is in contact with the tail end of the guiding membrane 250, and the guiding membrane 250 delivers a solution to the sample pad 260. Specifically, an upper surface of the tail end of the guiding membrane 250 is in contact with a lower surface of the front end of the sample pad 260, or, a lower surface of the tail end of the guiding membrane 250 is in contact with an upper surface of the front end of the sample pad 260.

Furthermore, the tail end of the sample pad 260 is in contact with the front end of the binding pad 240, and the sample pad 260 delivers a solution to the binding pad 240. Specifically, an upper surface of the tail end of the sample pad 260 is in contact with a lower surface of the front end of the binding pad 240, or, a lower surface of the tail end of the sample pad 260 is in contact with an upper surface of the front end of the binding pad 240.

In a third example of this embodiment, the immunochromatographic test strip 200 comprises two guiding membranes 250, which are respectively provided on the front end and the tail end of the sample pad 260. That is, a tail end of the first guiding membrane 250 is in contact with the front end of the sample pad 260, and the first guiding membrane 250 delivers a solution to the sample pad 260. Specifically, an upper surface of the tail end of the first guiding membrane 250 is in contact with the lower surface of the front end of the sample pad 260, or, a lower surface of the tail end of the first guiding membrane 250 is in contact with the upper surface of the front end of the sample pad 260.

The tail end of the sample pad 260 is in contact with the second guiding membrane 250, and the sample pad 260 delivers a solution to the second guiding membrane 250. Specifically, the upper surface of the tail end of the sample pad 260 is in contact with a lower surface of a front end of the second guiding membrane 250, or, the lower surface of the tail end of sample pad 260 is in contact with an upper surface of the front end of the second guiding membrane 250.

Wherein, in the case where there is only one guiding membrane 250, the guiding membrane 250 can be the first guiding membrane. In the case where there are two guiding membranes 250, the guiding membrane 250 near the chromatography membrane 220 can be the first guiding membrane, and the guiding membrane 250 away from the chromatography membrane 220 can be the second guiding membrane.

In this embodiment, the solution chromatography rate of the sample pad 260 is greater than the solution chromatography rate of the guiding membrane 250.

In this embodiment, the immunochromatographic test strip 200 has a length of 2 cm ~ 4 cm. Preferably, the length is 3.2 cm or 3.5 cm.

The use of this embodiment is substantially the same as that of embodiment 9, which will not be repeated here.

### Embodiment 11

This embodiment relates to the biochemical test strip placed in the biochemical test strip tube of embodiment 1 to embodiment 8.

In this embodiment, the biochemical test strip is illustrated as an immunochromatographic test strip.

As shown in FIG. 25 to FIG. 26, the immunochromatographic test strip 200 comprises a substrate 210, a chromatography membrane 220, an absorbent pad 230, a binding pad 240, a guiding membrane 250 and a sample limit membrane 270. Wherein, the sample limit membrane 270, the guiding membrane 250, the binding pad 240, the chromatography membrane 220 and the absorbent pad 230 are provided sequentially on the substrate 210.

Preferably, the structure, the connection and the composition of the substrate 210, the chromatography membrane 220, the absorbent pad 230 and the binding pad 240 are basically the same as in embodiment 9, which will not be repeated here.

The sample limit membrane 270 is provided on the upper surface of the substrate 210. That is, a lower surface of the sample limit membrane 270 is in contact with an upper surface of the substrate 210, such as by bonding. A tail end of the sample limit membrane 270 is in contact with a front end of the guiding membrane 250, and the sample limit membrane 270 delivers a solution to the guiding membrane 250. Specifically, an upper surface of the tail end of the sample limit membrane 270 is in contact with a lower surface of the front end of the guiding membrane 250, or, a lower surface of the tail end of the sample limit membrane 270 is in contact with an upper surface of the front end of the guiding membrane 250.

Preferably, the sample limit membrane 270 is capable of absorbing small amounts of sample with an aspiration saturation volume of 2~20 µl.

Preferably, the sample limit membrane 270 is made of glass fiber, polyester film, cellulose filter paper and so on.

In this embodiment, the immunochromatographic test strip 200 has a length of 2 cm ~ 4 cm. Preferably, the length is 3.3 cm, 3.5 cm or 3.7 cm.

### Embodiment 12

This embodiment relates to the biochemical test strip placed in the biochemical test strip tube of embodiment 1 to embodiment 8.

In this embodiment, the biochemical test strip is illustrated as an immunochromatographic test strip.

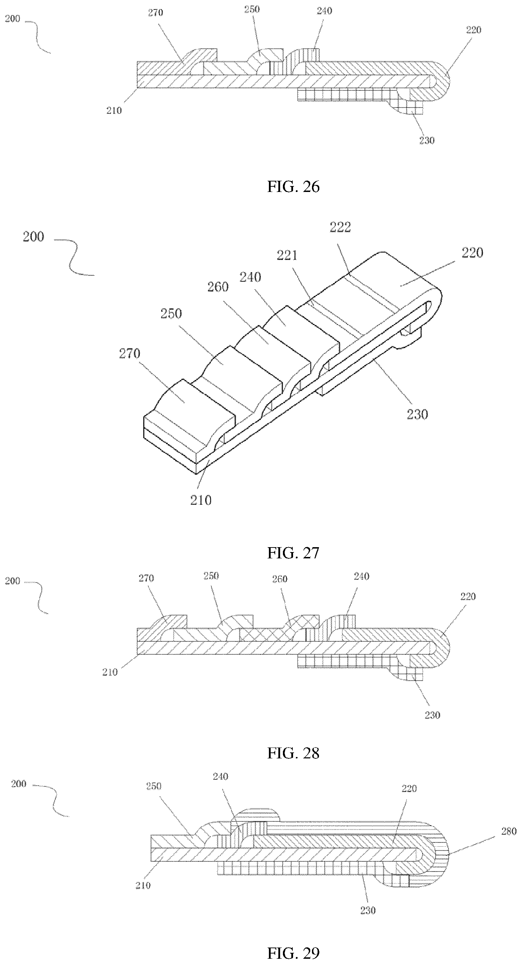

As shown in FIG. 27 to FIG. 28, the immunochromatographic test strip 200 comprises a substrate 210, a chromatography membrane 220, an absorbent pad 230, a binding pad 240, a guiding membrane 250, a sample pad 260 and a sample limit membrane 270. Wherein, the sample limit membrane 270, the guiding membrane 250, the sample pad 260, the binding pad 240, the chromatography membrane 220 and the absorbent pad 230 are provided sequentially on the substrate 210.

Preferably, the structure, the connection and the composition of the substrate 210, the chromatography membrane 220, the absorbent pad 230, the binding pad 240, the guiding membrane 250 and the sample pad 260 are basically the same as the second example and the third example in embodiment 10, which will not be repeated here.

The sample limit membrane 270 is provided on the upper surface of the substrate 210. That is, a lower surface of the sample limit membrane 270 is in contact with an upper surface of the substrate 210, such as by bonding. A tail end of the sample limit membrane 270 is in contact with a front end of the guiding membrane 250, and the sample limit membrane 270 delivers a solution to the guiding membrane 250. Specifically, an upper surface of the tail end of the sample limit membrane 270 is in contact with a lower surface of the front end of the guiding membrane 250, or, a lower surface of the tail end of the sample limit membrane 270 is in contact with an upper surface of the front end of the guiding membrane 250.

Preferably, the sample limit membrane 270 is capable of absorbing small amounts of sample with an aspiration saturation volume of 2~20 µl.

Preferably, the sample limit membrane 270 is made of glass fiber, polyester film, cellulose filter paper and so on.

In this embodiment, the immunochromatographic test strip 200 has a length of 2 cm ~ 4 cm. Preferably, the length is 3.6 cm or 3.8 cm.

### Embodiment 13

This embodiment relates to the biochemical test strip placed in the biochemical test strip tube of embodiment 1 to embodiment 8.

In this embodiment, the biochemical test strip is illustrated as an immunochromatographic test strip.

As shown in FIG. 29, the immunochromatographic test strip 200 comprises a substrate 210, a chromatography membrane 220, an absorbent pad 230, a binding pad 240, a guiding membrane 250 and a transparent protective film 280. Wherein, the guiding membrane 250, the binding pad 240, the chromatography membrane 220 and the absorbent pad 230 are provided sequentially on the substrate 210.

Preferably, the structure, the connection and the composition of the substrate 210, the chromatography membrane 220, the absorbent pad 230, the binding pad 240 and the guiding membrane 250 are basically the same as in embodiment 9, which will not be repeated here.

A lower surface of the transparent protective film 280 covers at least the binding pad 240 and the chromatography membrane 220.

In this embodiment, the use of the transparent protective film 280 can prevent the binding pad 240 and the chromatography membrane 220 from contacting an exogenous water-containing mixture and improve the correctness of the detection results of the immunochromatographic test strip 200.

In addition, the transparent protective film 280 can also be applied to the immunochromatographic test strip 200 of embodiment 9 to embodiment 12.

### Embodiment 14

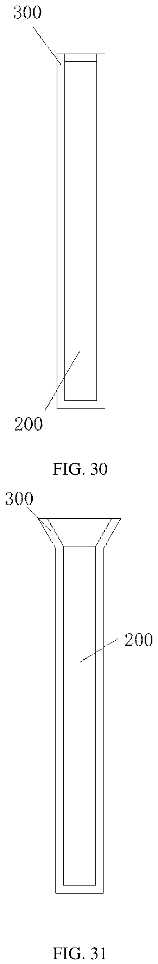

As shown in FIG. 30, the immunochromatographic test strip 200 further comprises a transparent hollow tube 300. Wherein, the transparent hollow tube 300 wraps the immunochromatographic test strip 200 so that the immunochromatographic test strip 200 is located inside the transparent hollow tube 300. That is, the immunochromatographic test strip 200 is provided inside the transparent hollow tube 300.

The inner surface of the transparent hollow tube 300 is a hydrophobic surface.

A first end of the transparent hollow tube 300 is a closed end, and a second end of the transparent hollow tube 300 is an open end. The purpose of providing the open end is to facilitate the movement of the immunochromatographic test strip 200 from an opening to the interior of the transparent hollow tube 300.

In addition, the sample solution receiving end of the immunochromatographic test strip 200 is located at the open end of the transparent hollow tube 300.

Preferably, the transparent hollow tube 300 is made of transparent material, such as plastic, thermoplastic resin, to facilitate observation of the test or diagnostic results of the immunochromatographic test strip 200.

In one example of this embodiment, as shown in FIG. 31, a width of the cross-section of a second end of the open end of the transparent hollow tube 300 is greater than the inner diameter of the cross-section of a first end of the open end of the transparent hollow tube 300. Preferably, the width of the cross-section of the open end of the transparent hollow tube 300 decreases from the second end of the open end to the first end of the open end.

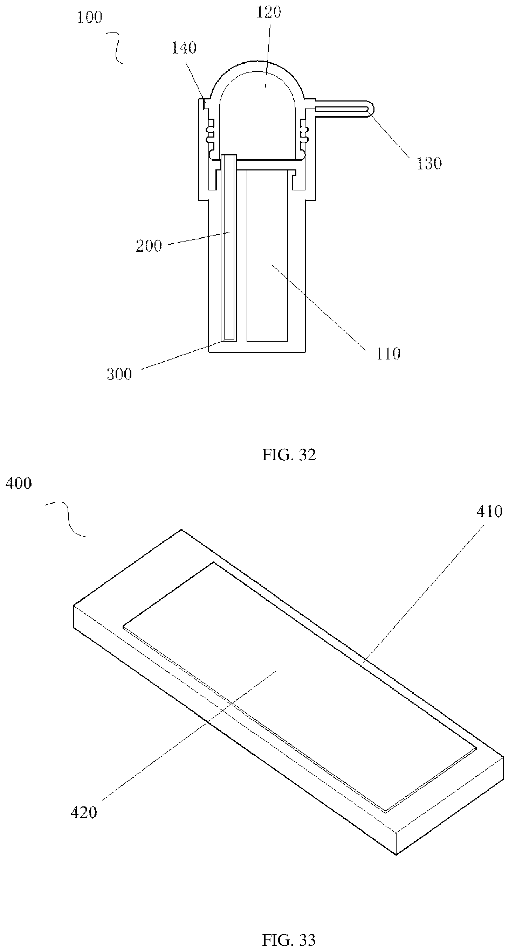

As shown in FIG. 32, the immunochromatographic test strip 200 with the transparent hollow tube 300 is placed into the test strip fixing member 112 of the biochemical test strip tube 100 to avoid the movement of the immunochromatographic test strip 200 within the test strip fixing member 112.

In addition, the use of the transparent hollow tube 300 can prevent the immunochromatographic test strip 200 from directly contacting the inner surface of the test strip fixing member 112 and prevent the surface tension of the inner surface of the test strip fixing member 112 from affecting the adsorption rate of the immunochromatographic test strip 200.

### Embodiment 15

This embodiment relates to the test strip fixing member the biochemical test strip tube of embodiment 1 to embodiment 8.

In this embodiment, the test strip fixing member is illustrated as a test strip cassette.

As shown in FIG. 33 to FIG. 34, the test strip cassette 400 comprises a bottom card 410 and a first sealing member 420. Wherein, the first sealing member 420 covers a first end surface of the bottom card 410 for sealing the biochemical test strip located inside the bottom card 410.

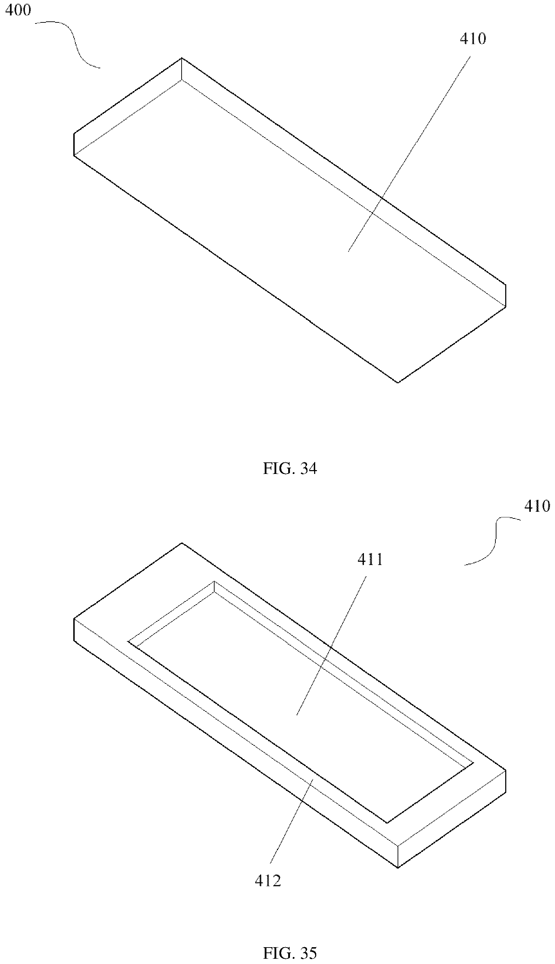

As shown in FIG. 35, the bottom card 410 comprises a biochemical test strip slot 411 and a first sealing surface 412. Wherein, the biochemical test strip slot 411 is provided on the first end surface of the bottom card 410 for placing the biochemical test strip, and the first sealing surface 412 is provided around the biochemical test strip slot 411 for bonding the first sealing member 420.

In one example of this embodiment, the bottom card 410 is made of transparent material, such as plastic, to facilitate observation of the test process.

The first sealing member 420 is bonded to the first sealing surface 412, which makes the biochemical test strip slot 411 form a sealed space.

In one example of this embodiment, the first sealing member 420 is a first sealing film, and comprises at least an aluminum foil film.

In this embodiment, the biochemical test strip is illustrated as an immunochromatographic test strip.

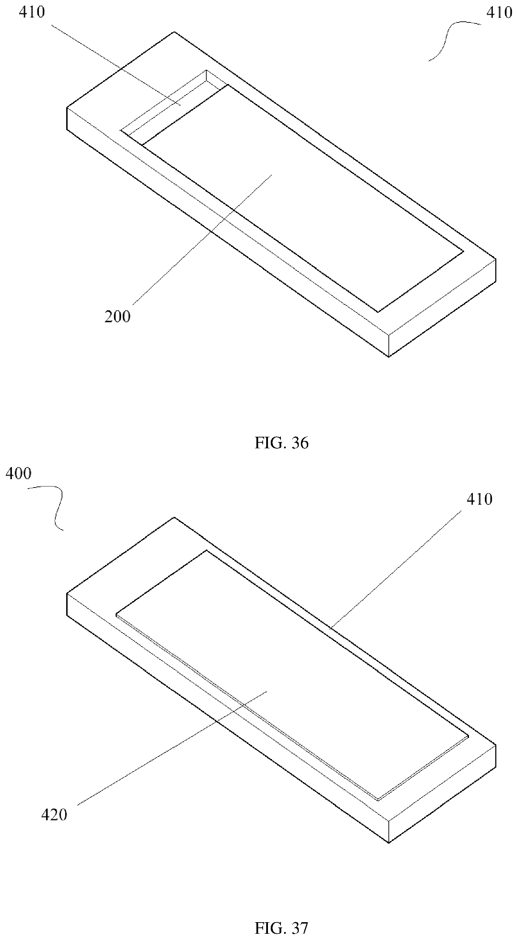

As shown in FIG. 36, the immunochromatographic test strip 200 is provided inside the biochemical test strip slot 411, and the length of the immunochromatographic test strip 200 is less than a length of the biochemical test strip slot 411.

This embodiment is used as follows: tearing off the first sealing member 420 along one end; adding a sample solution to the sample receiving end of the immunochromatographic test strip 200 located in the biochemical test strip slot 411, and then waiting for observation.

### Embodiment 16

This embodiment is a variation of embodiment 15. As shown in FIG. 37 to FIG. 38, a test strip cassette 400 comprises a bottom card 410, a first sealing member 420 and a second sealing member 430. Wherein, the first sealing member 420 covers a first end surface of the bottom card 410, and the second sealing member 430 covers a second end surface of the bottom card 410. The first sealing member 420 and the second sealing member 430 are used to keep the biochemical test strip provided inside the bottom card 410 to be inside a confined space, thereby keeping the biochemical test strip dry.

Preferably, the structure and the connection of the first sealing member 420 is basically the same as in embodiment 15, which will not be repeated here.

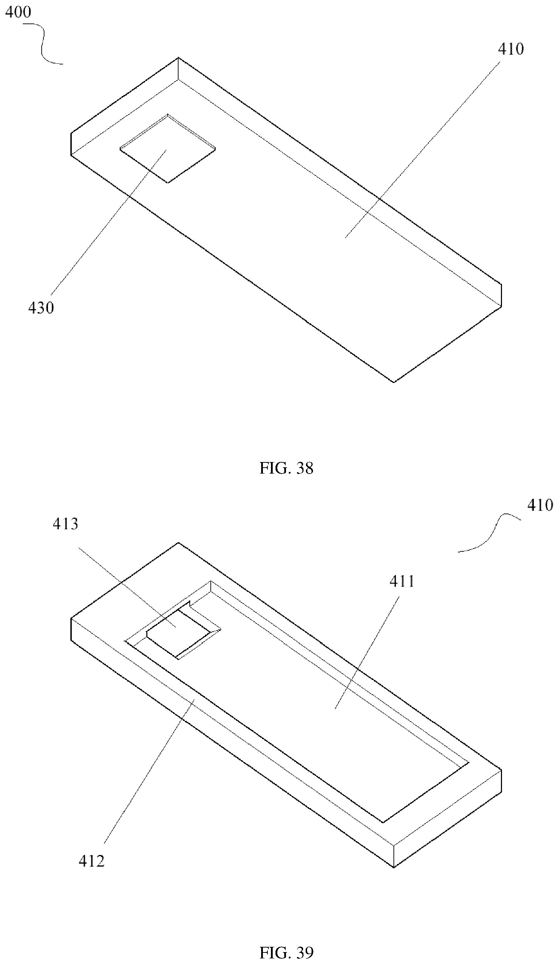

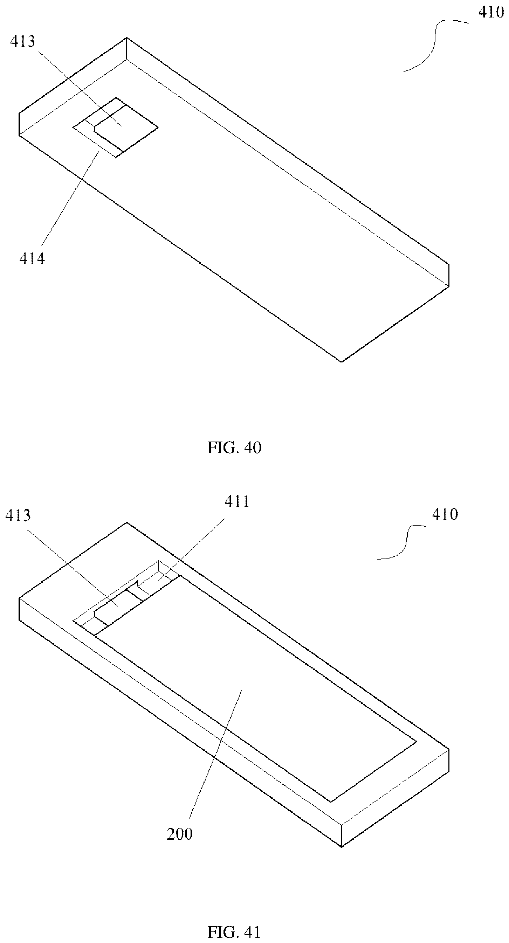

As shown in FIG. 39 to FIG. 40, the bottom card 410 comprises a biochemical test strip slot 411, a first sealing surface 412, a sample solution addition hole 413 and a second sealing surface 414. Wherein, the biochemical test strip slot 411 is provided on the first end surface of the bottom card 410 for placing the biochemical test strip. The first sealing surface 412 is provided around the biochemical test strip slot 411 for bonding the first sealing member 420. The sample solution addition hole 413 is provided on the second end surface of the bottom card 410 (the second end surface is set opposite to the first end surface, that is, the first end surface is an upper surface and the second end surface is a lower surface) and communicates with the biochemical test strip slot 411 for adding a sample solution to the biochemical test strip inside the biochemical test strip slot 411 by the sample solution addition hole 413. The second sealing surface 414 is provided around the sample solution addition hole 413 for bonding the second sealing member 430.

Wherein, a length of the sample solution addition hole 413 is less than the length of the biochemical test strip slot 411. In addition, the sample solution addition hole 413 is staggered with the biochemical test strip slot 411. That is, a lower portion of the sample solution addition hole 413 communicates with an upper portion of the biochemical test strip slot 411.

In one example of this embodiment, a width of the sample solution addition hole 413 is less than the width of the biochemical test strip slot 411.

The second sealing member 430 is bonded to the second sealing surface 413 for closing the sample solution addition hole 413, which makes the interior of the bottom card 410 form a sealed space that keeps the biochemical test strip located inside the biochemical test strip slot 411 in a dry environment.

In one example of this embodiment, the second sealing member 430 is a second sealing film, and comprises at least an aluminum foil film.

In this embodiment, the biochemical test strip is illustrated as an immunochromatographic test strip.

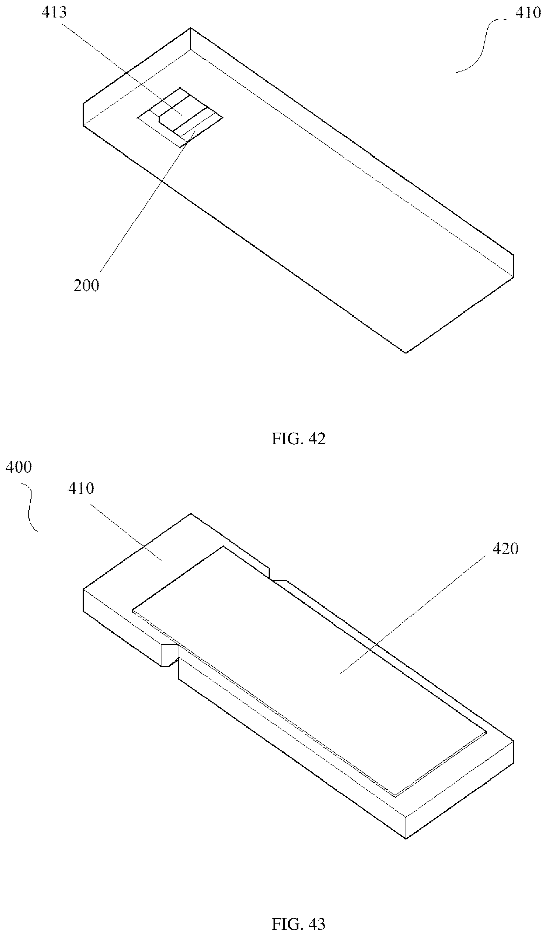

As shown in FIG. 41 to FIG. 42, the immunochromatographic test strip 200 is provided inside the biochemical test strip slot 411, and the sample receiving end of the immunochromatographic test strip 200 is located at the connection between the sample solution addition hole 413 and the biochemical test strip slot 411 to make the sample solution contact with the immunochromatographic test strip 200 directly.

This embodiment is used as follows: removing the second sealing member 430; adding a sample solution to the sample receiving end of the immunochromatographic test strip 200 located in the biochemical test strip slot 411 by the sample solution addition hole 413, and then waiting for observation.

### Embodiment 17

This embodiment is a variation of embodiment 16. As shown in FIG. 43 to FIG. 44, a test strip cassette 400 comprises a bottom card 410, a first sealing member 420 and a second sealing member 430. Wherein, the first sealing member 420 covers a first end surface of the bottom card 410, and the second sealing member 430 covers a second end surface of the bottom card 410. The first sealing member 420 and the second sealing member 430 are used to keep the biochemical test strip provided inside the bottom card 410 to be inside a confined space, thereby keeping the biochemical test strip dry.

Preferably, the structure and the connection of the first sealing member 420 and the second sealing member 430 are basically the same as in embodiment 16, which will not be repeated here.

In this embodiment, the biochemical test strip is illustrated as an immunochromatographic test strip.

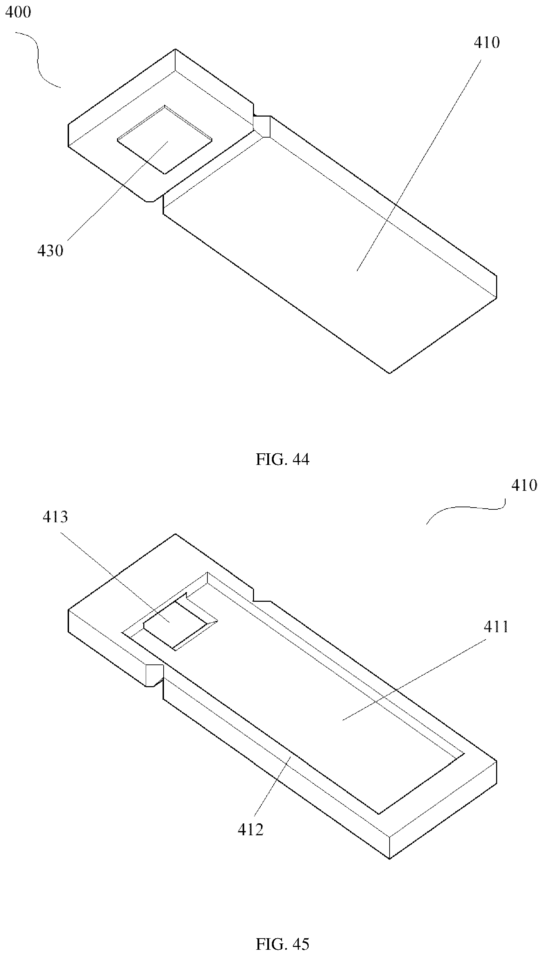

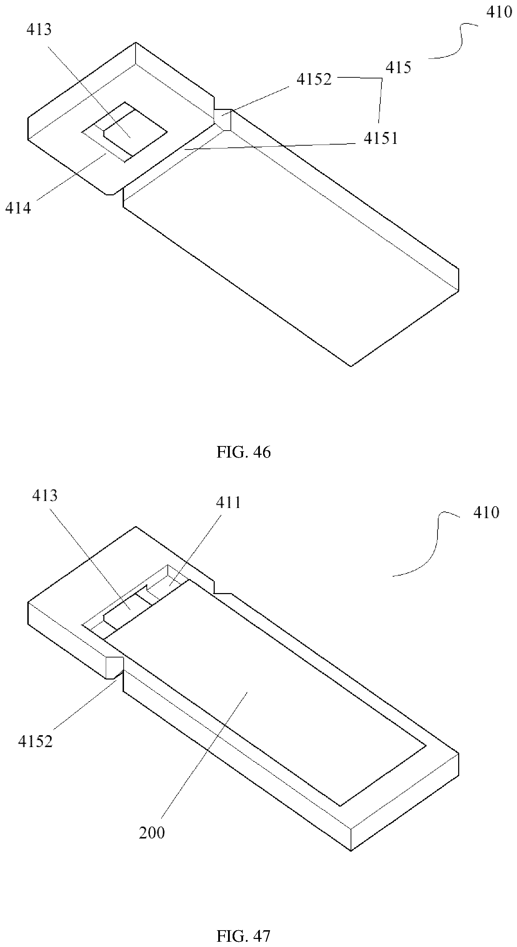

As shown in FIG. 45 to FIG. 48, the bottom card 410 comprises a biochemical test strip slot 411, a first sealing surface 412, a sample solution addition hole 413, a second sealing surface 414 and a first creasing member 415.

Preferably, the structure and the connection of the biochemical test strip slot 411, the first sealing surface 412, the sample solution addition hole 413, and the second sealing surface 414 are basically the same as in embodiment 16, which will not be repeated here.

The first creasing member 415 is provided on the second end surface of the bottom card 410 and near the sample solution addition hole 413 for bending the bottom card 410 at the first creasing member 415 to expose the sample solution receiving end of the immunochromatographic test strip 200 located inside the bottom card 410 to the bent cross section of the first creasing member 415.

The first creasing member 415 comprises a first creasing element 4151 and a second creasing element 4152. Wherein, the first creasing element 4151 is provided on the second end surface of the bottom card 410 and near the sample solution addition hole 413. The second creasing element 4152 is provided symmetrically at both ends of the first creasing element 4151 (that is, the second creasing element 4152 is provided on a third end surface (left side) and a fourth end surface (right side) of the bottom card 410) and connected to the first creasing element 4151.

In one example of this embodiment, the first creasing element 4151 is a horizontal crease, and the second creasing element 4152 is a vertical crease which allows one end of the bottom card 410 to be quickly bent at the first creasing member 415.

In one example of this embodiment, after bending the bottom card 410 along the first creasing member 415, the sample solution receiving end of the immunochromatographic test strip 200 provided inside the biochemical test strip slot 411 extends for a length greater than 0.5 mm outside of the biochemical test strip slot 411.

In one example of this embodiment, the immunochromatographic test strip 200 provided inside the biochemical test strip slot 411 is provided with a cavity at an outer end of an intersection of the first creasing member 415 and the immunochromatographic test strip 200 to avoid the immunochromatographic test strip 200 being bent when the bottom card 410 is bent.

In one example of this embodiment, a ratio of a height of the cavity to a length of the outer end of the immunochromatographic test strip 200 is greater than 70%.

### Embodiment 18

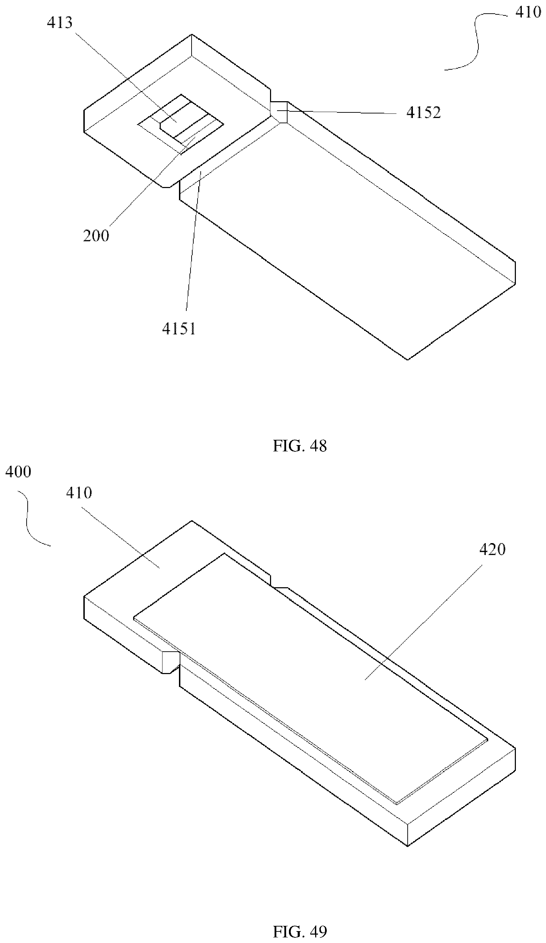

This embodiment is a variation of embodiment 17. As shown in FIG. 49 to FIG. 50, a test strip cassette 400 comprises a bottom card 410, a first sealing member 420 and a second sealing member 430. Wherein, the first sealing member 420 covers a first end surface of the bottom card 410, and the second sealing member 430 covers a second end surface of the bottom card 410. The first sealing member 420 and the second sealing member 430 are used to keep the biochemical test strip provided inside the bottom card 410 to be inside a confined space, thereby keeping the biochemical test strip dry.

Preferably, the structure and the connection of the first sealing member 420 and the second sealing member 430 are basically the same as in embodiment 17, which will not be repeated here.

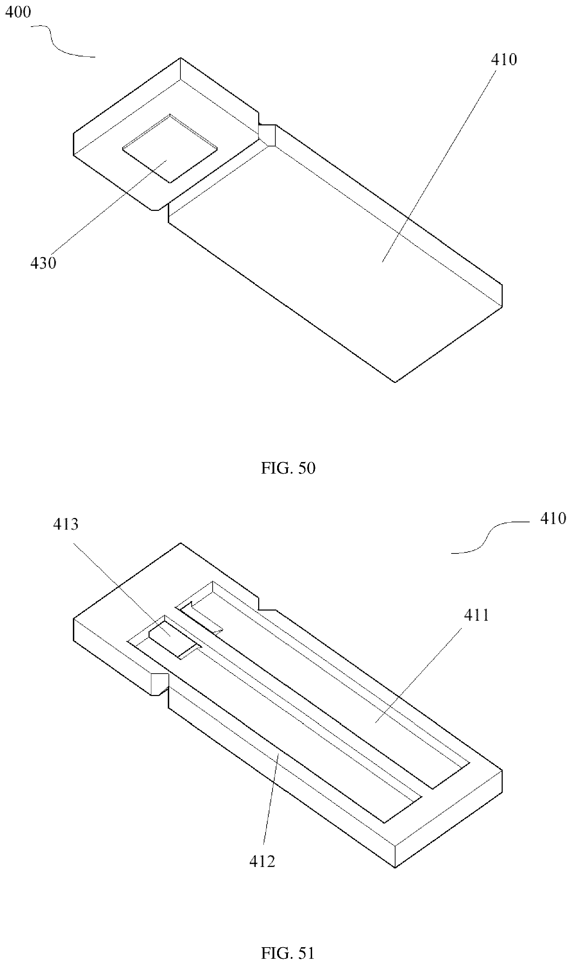

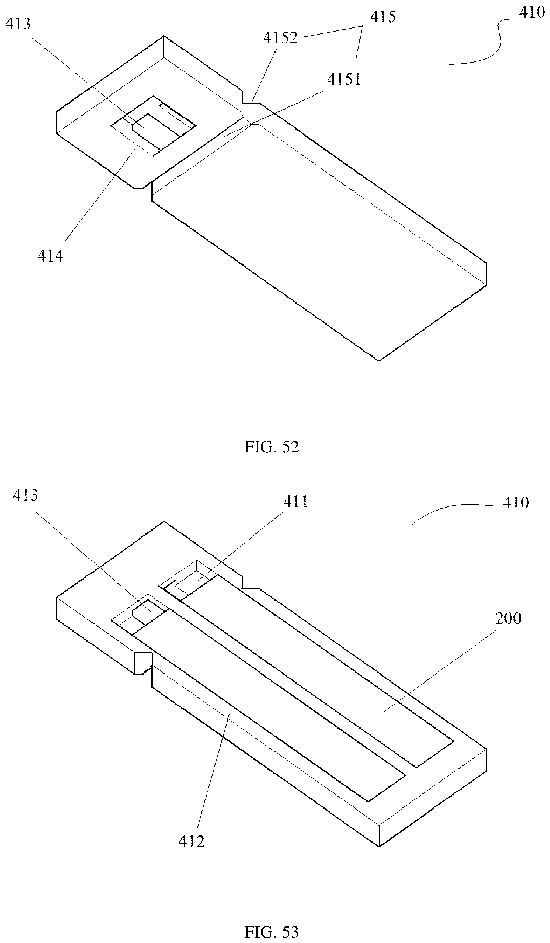

As shown in FIG. 51 to FIG. 54, the bottom card 410 comprises a biochemical test strip slot 411, a first sealing surface 412, a sample solution addition hole 413, a second sealing surface 414 and a first creasing member 415.

Preferably, the structure and the connection of the first sealing surface 412, the sample solution addition hole 413, the second sealing surface 414, and the first creasing member 415 are basically the same as in embodiment 17, which will not be repeated here.

The number of the biochemical test strip slot 411 is two. The biochemical test strip slots 411 are provided side by side. The biochemical test strip slots 411 are spaced apart so that the immunochromatographic test strips 200 located in each biochemical test strip slot 411 are not in contact with each other. The biochemical test strip slots 411 are each communicated to the sample solution addition hole 413 respectively.

In one example of this embodiment, the number of the biochemical test strip slots 411 may be more than two.

In this embodiment, the purpose of providing two biochemical test strip slots 411 is to allow different test to be performed simultaneously.

### Embodiment 19

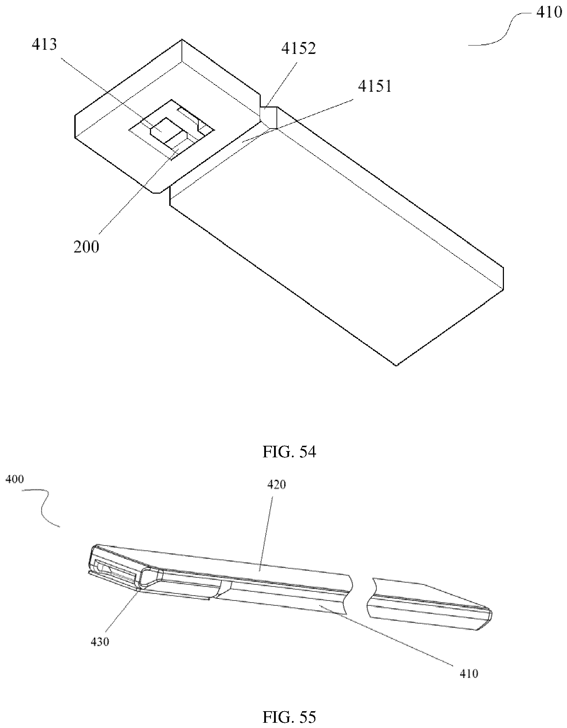

This embodiment is a variation of embodiment 18. As shown in FIG. 55, a test strip cassette 400 comprises a bottom card 410, a first sealing member 420 and a second sealing member 430. Wherein, the first sealing member 420 covers a first end surface of the bottom card 410, and the second sealing member 430 covers a second end surface of the bottom card 410. The first sealing member 420 and the second sealing member 430 are used to keep the biochemical test strip provided inside the bottom card 410 to be inside a confined space, thereby keeping the biochemical test strip dry.

Preferably, the structure and the connection of the first sealing member 420 and the second sealing member 430 are basically the same as in embodiment 18, which will not be repeated here.

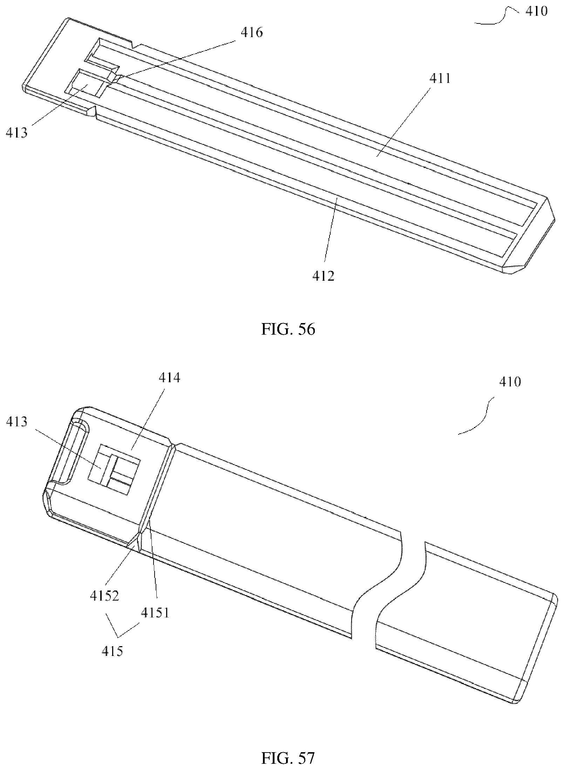

As shown in FIG. 56 to FIG. 58, the bottom card 410 comprises a biochemical test strip slot 411, a first sealing surface 412, a sample solution addition hole 413, a second sealing surface 414, a first creasing member 415 and a second creasing member 416.

Preferably, the structure and the connection of the biochemical test strip slot 411, the first sealing surface 412, the sample solution addition hole 413, the second sealing surface 414, and the first creasing member 415 are basically the same as in embodiment 18, which will not be repeated here.

The second creasing member 416 is provided on the first end surface of the bottom card 410 and is located inside the biochemical test strip slot 411 corresponding to the first creasing member 415, thereby the bottom card 410 can be bent quickly.

In one example of this embodiment, the second creasing member 416 is a horizontal crease.

### Embodiment 20

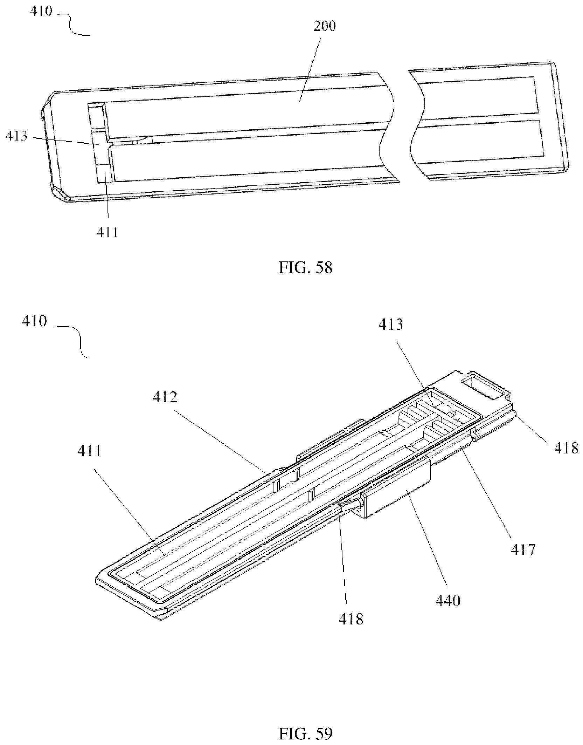

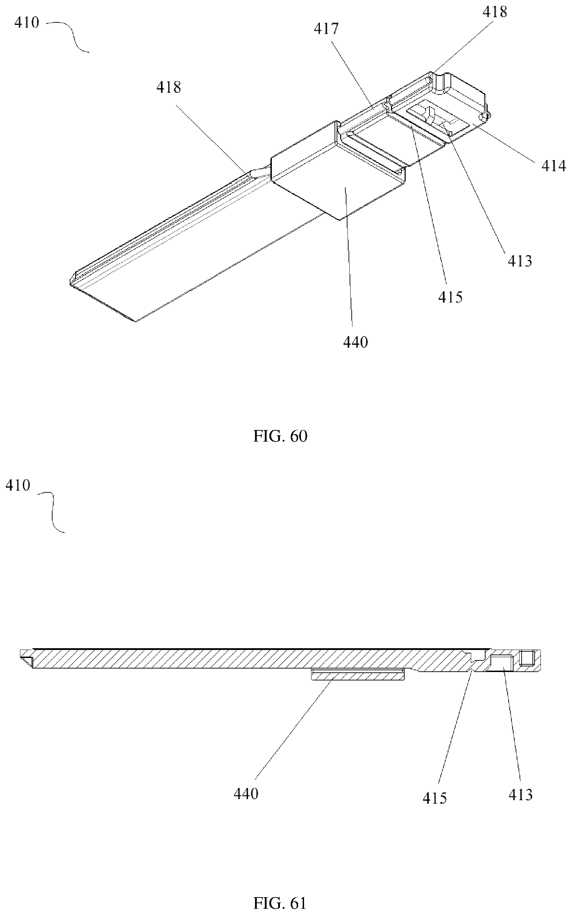

This embodiment is a variation of embodiment 19. As shown in FIG. 59 to FIG. 61, a test strip cassette 400 comprises a bottom card 410, a first sealing member 420, a second sealing member 430 and a sliding member 440. Wherein, the first sealing member 420 covers a first end surface of the bottom card 410, and the second sealing member 430 covers a second end surface of the bottom card 410. The first sealing member 420 and the second sealing member 430 are used to keep the biochemical test strip provided inside the bottom card 410 to be inside a confined space, thereby keeping the biochemical test strip dry. The sliding member 440 is slidingly provided on the bottom card 410 for closing the bottom card 410 in a particular case.

Preferably, the structure and the connection of the first sealing member 420 and the second sealing member 430 are basically the same as in embodiment 19, which will not be repeated here.

The bottom card 410 comprises a biochemical test strip slot 411, a first sealing surface 412, a sample solution addition hole 413, a second sealing surface 414, a first creasing member 415, a second creasing member 416, a guide member 417 and an anti-releasing member 418.

Preferably, the structure and the connection of the biochemical test strip slot 411, the first sealing surface 412, the sample solution addition hole 413, the second sealing surface 414, the first creasing member 415 and second creasing member 416 are basically the same as in embodiment 19, which will not be repeated here.

The guide member 417 is provided symmetrically on the left side and the right side of the bottom card 410 for sliding connection with the sliding member 440, which allows the sliding member 440 to reciprocate along the guide member 417.

Wherein, the guide member 417 is provided across the first creasing member 415. Specifically, after bending the bottom card 410 at the first creasing member 415, the bottom card 410 is divided into a first part and a second part, the first part of the bottom card 410 is not separated from the second part of the bottom card 410, and the first part of the bottom card 410 and the second part of the bottom card 410 are in a bent state. The guide member 417 is provided on the left side and the right side of the first part and the second part of the bottom card 410, at which time, the sliding member 440 is located in the first part of the bottom card 410. After the second part of the bottom card 410 is reset, the second part of the bottom card 410 and the first part of the bottom card 410 are in a horizontal state, at which time, the sliding member 440 slides along the guide member 417 with one portion of the sliding member 440 slidingly connected to the guide member 417 of the first part of the bottom card 410 and the other portion of the sliding member 440 slidingly connected to the second part of the bottom card 410, and the sliding member 440 obscures the sample solution addition hole 413 to seal the bottom card 410.

Wherein, a first end of the guide member 417 is provided on the first end of the bottom card 410 (that is, near the sample solution addition hole 413) and a second end of the guide member 417 is provided on the middle of the bottom card 410 for reducing the sliding distance of the sliding member 440.

The anti-releasing member 418 is provided on both ends of the guide member 417 for preventing the sliding member 440 from falling off.

In one example of this embodiment, the anti-releasing member 418 is a limit stopper or limit projection.

This embodiment is used as follows: bending the bottom card 410 at the first creasing member 415 and the second creasing member 416, at which time the bottom card 410 is divided into the first part and the second part, to expose the immunochromatographic test strip 200 provided inside the biochemical test strip slot 411 of the bottom card 410, so that the sample solution receiving end of the immunochromatographic test strip 200 contacts the sample solution; resetting the second part of the bottom card 410 to make the second part of the bottom card 410 is in a horizontal state again; sliding the sliding member 440 along the guide member 417 with one portion of the sliding member 440 slidingly connected to the guide member 417 of the first part of the bottom card 410 and the other portion of the sliding member 440 slidingly connected to the second part of the bottom card 410, and the sliding member 440 obscures the sample solution addition hole 413 to seal the bottom card 410, thereby creating a brief airtight state to avoid outer water vapor from contacting the immunochromatographic test strip 200.

The above embodiments are merely illustrative of several implementation manners of the present disclosure, and the description thereof is more specific and detailed, but is not to be construed as a limitation to the patentable scope of the present disclosure. It should be pointed out that several variations and improvements can be made by those of ordinary skill in the art without departing from the conception of the present disclosure, but such variations and improvements should fall within the protection scope of the present disclosure. Therefore, the protection scope of the patent of the present disclosure should be subject to the appended claims.

## Claims

1. A biochemical test strip tube, wherein, the biochemical test strip tube (100) comprising a tube portion (110) and a cover portion (120);
the tube portion (110) comprises:
a solution chamber (111);
a test strip fixing member (112), in which, when the cover portion (120) seals off the tube portion (110), the test strip fixing member (112) or a top of a sample receiving end of a biochemical test strip located within the test strip fixing member (112) will extend to a bottom end of an inner wall of the cover portion (120), with a vertical spacing of less than 2 mm from a top of the cover portion (120), and the test strip fixing member (112) has a horizontal spacing between an outer edge of the test strip fixing member (112) and an inner wall of a mouth of the tube portion (110), and the test strip fixing member (112) or the top of the sample receiving end of the biochemical test strip located within the test strip fixing member (112) will project at least 1 mm above the level of the mouth of the tube portion (110); and
the cover portion (120) comprises:
a cavity (122) formed by an inner surface of the cover portion (120) projecting outwardly.

2. The biochemical test strip tube according to claim 1, wherein, the test strip fixing member (112) has a width of not more than 2.8 mm, and has a depth of not less than 28 mm.

3. The biochemical test strip tube according to claim 1 or claim 2, wherein, the horizontal spacing is greater than a wall thickness of an inner insertion port of the cover portion (120).

4. The biochemical test strip tube according to any one of claim 1 to claim 3, wherein, the test strip fixing member (112) is a biochemical test strip chamber, and a top of the biochemical test strip chamber communicates with the solution chamber (111); or
the test strip fixing member (112) is a biochemical test strip container positioned by a snap-in side wall (113) inside the solution chamber (111) and an outer wall of the solution chamber (111), and the biochemical test strip container is provided with a connecting hole at a top of the biochemical test strip container which is connected to the sample solution receiving end of the biochemical test strip, or the biochemical test strip located inside the biochemical test strip container extends a specified length from the top of the biochemical test strip container; or
the test strip fixing member (112) is a biochemical test strip container positioned by a snap-in side wall (113) inside the solution chamber (111) and an outer wall of the solution chamber (111), in which, after a top end of the biochemical test strip container is folded, a fractured cross-section of the biochemical test strip container has a connecting hole connected to the sample solution receiving end of the biochemical test strip, or the biochemical test strip located inside the biochemical test strip container extends a specified length from the top of the biochemical test strip container.

5. The biochemical test strip tube according to any one of claim 1 to claim 4, wherein, the cover portion (120) further comprises:
a limit member (121) provided on an outer wall of the cover portion (120); and
the biochemical test strip tube (100) further comprises:
an anti-opening member (140) provided on the top of the tube portion (110) and/or provided on the limit member (121) of the cover portion (120).

6. The biochemical test strip tube according to claim 5, wherein, the anti-opening member (140) comprises:
a countersink hole (141) provided on a top of the mouth of the tube portion (110).

7. The biochemical test strip tube according to claim 5 or claim 6, wherein, the anti-opening member (140) comprises:
a chamfered member (142) provided on an outer wall of the limit member (121), when the cover portion (120) seals off the tube portion (110), a width of a cross section of an upper end of the chamfered member (142) is less than a width of a cross section of a lower end of the chamfered member (142).

8. The biochemical test strip tube according to claim 6, wherein, the anti-opening member (140) further comprises:
an opening member (143) provided on the countersink hole (141), in which, a first end of the opening member (143) is located on an inner wall of the countersink hole (141), and a second end of the opening member (143) is located on an outer wall of the countersink hole (141) or between the outer wall of the countersink hole (141) and the inner wall of the countersink hole (141).

9. The biochemical test strip tube according to any one of claim 1 to claim 8, wherein, the cover portion (120) further comprises:
a sealing member (123) provided on an outer wall of the cover portion (120);
a first fitting member (124) provided on the outer wall of the cover portion (120) and located above the sealing member (123); and
the tube portion (110) further comprises:
a second fitting member (114) provided on an inner wall of the tube portion (110), and the second fitting member (114) can be nested and connected to the first fitting member (124).

10. The biochemical test strip tube according to any one of claim 1 to claim 9, wherein, the biochemical test strip tube (100) further comprises:
a connecting member (130) connected to the tube portion (110) and the cover portion (120) respectively.

11. The biochemical test strip tube according to any one of claim 1 to claim 10, wherein, the biochemical test strip tube (100) further comprises:
a biochemical test strip provided inside the test strip fixing member (112), in which, when the cover portion (120) seals off the tube portion (110), the sample receiving end of the biochemical test strip is proximate to the cover portion (120), and a horizontal spacing is provided between the sample receiving end of the biochemical test strip and the inner wall of the cover portion (120).

12. The biochemical test strip tube according to claim 11, wherein, the biochemical test strip protrudes from the test strip fixing member (112).

13. The biochemical test strip tube according to claim 11 or claim 12, wherein, the biochemical test strip is an immunochromatographic test strip (200) which comprises:
a substrate (210);
a chromatography membrane (220) provided on an upper surface of the substrate (210);
an absorbent pad (230) provided on a lower surface of the substrate (210); and
the chromatography membrane (220) and the absorbent pad (230) are in contact at a tail end of the substrate (210) and perform solution transfer.

14. The biochemical test strip tube according to claim 13, wherein, a tail end of the chromatography membrane (220) extends a specified distance beyond the tail end of the substrate (210), a tail end of the absorbent pad (230) extends a specified distance beyond the tail end of the substrate (210), and the tail end of the chromatography membrane (220) and the tail end of the absorbent pad (230) are in contact and perform solution transfer; or
the chromatography membrane (220) is also provided on the lower surface of the substrate (210), and the tail end of the chromatography membrane (220) and the tail end of the absorbent pad (230) are in contact on the lower surface of the substrate (210) and perform solution transfer; or
the absorbent pad (230) is also provided on the upper surface of the substrate (210), and the tail end of the chromatography membrane (220) and the tail end of the absorbent pad (230) are in contact on the upper surface of the substrate (210) and perform solution transfer.

15. The biochemical test strip tube according to claim 13 or claim 14, wherein, the immunochromatographic test strip (200) further comprises:
a binding pad (240) provided on the substrate (210), in which a tail end of the binding pad (240) is in contact with a front end of the chromatography membrane (220), and the binding pad (240) delivers a solution to the chromatography membrane (220);
a guiding membrane (250) provided on the substrate (210), in which a tail end of the guiding membrane (250) is in contact with a front end of the binding pad (240), and the guiding membrane (250) delivers a solution to the binding pad (240); and
in which, the solution chromatography rate of the guiding membrane (250) is less than the solution chromatography rate of the binding pad (240).

16. The biochemical test strip tube according to any one of claim 11 to claim 15, wherein, the biochemical test strip tube (100) further comprises:
a transparent hollow tube (300) provided inside the test strip fixing member (112), and the transparent hollow tube (300) is provided with the biochemical test strip.

17. The biochemical test strip tube according to claim 16, wherein, one end of the transparent hollow tube (300) is a closed end.

18. The biochemical test strip tube according to any one of claim 1 to claim 17, wherein, the test strip fixing member (112) is a test strip cassette (400) which comprises:
a bottom card (410) which comprises:
a biochemical test strip slot (411) provided on a first end surface of the bottom card (410) for placing the biochemical test strip;
a first sealing surface (412) provided around the biochemical test strip slot (411); and
a first sealing member (420) bonded to the first sealing surface (412), which makes the biochemical test strip slot (411) form a sealed space.

19. The biochemical test strip tube according to claim 18, wherein, the bottom card (410) further comprises:
a sample solution addition hole (413) provided on a second end surface of the bottom card (410), and connected to the biochemical test strip slot (411);
a second sealing surface (414) provided around the sample solution addition hole (413); and
the test strip cassette (400) further comprises:
a second sealing member (430) bonded to the second sealing surface (414), which makes the sample solution addition hole (413) form a sealed space.

20. The biochemical test strip tube according to claim 19, wherein, the bottom card (410) further comprises:
a first creasing member (415) provided on the second end surface of the bottom card (410) and provided close to the sample solution addition hole (413); and
in which, after folding the bottom card (410) along the first creasing member (415), the sample solution receiving end of the biochemical test strip provided inside the biochemical test strip slot (411) is expose to a folded section of the first creasing member (415).

21. The biochemical test strip tube according to claim 20, wherein, after folding the bottom card (410) along the first creasing member (415), the sample solution receiving end of the biochemical test strip provided inside the biochemical test strip slot (411) extends for a length greater than 0.5 mm outside of the biochemical test strip slot (411).

22. The biochemical test strip tube according to claim 20 or claim 21, wherein, the first creasing member (415) comprises:
a first creasing element (4151) provided on the second end surface of the bottom card (410) and provided close to the sample solution addition hole (413); and
a second creasing element (4152) provided symmetrically at both ends of the first creasing element (4151) and connected to the first creasing element (4151).

23. The biochemical test strip tube according to any one of claim 20 to claim 22, wherein, the biochemical test strip provided inside the biochemical test strip slot (411) is provided with a cavity at an outer end of an intersection of the first creasing member (415) and the biochemical test strip, and a ratio of a height of the cavity to a length of the outer end of the biochemical test strip is greater than 70%.

24. The biochemical test strip tube according to any one of claim 20 to claim 23, wherein, the bottom card (410) further comprises:
a guide member (417) provided on both sides of the bottom card (410) and extended from one side of the first creasing member (415) to the other side of the first creasing member (415);
the test strip cassette (400) further comprises:
a sliding member (440) slidably connected to the guide member (417);
in which, after bending the bottom card (410) along the first creasing member (415), the bottom card (410) is divided into a first part and a second part, both the first part and the second part are in a bent state, and the slide member is initially located in the first part; and
after the second part has been reset, both the first part and the second part are in a horizontal state, one end of the sliding member (440) is located in the first part, the other end of the sliding member (440) is located in the second part, and the sliding member (440) seals the first creasing member (415).

25. The biochemical test strip tube according to claim 24, wherein, the bottom card (410) further comprises:
an anti-releasing member (418) provided on both ends of the guide member (417) for limiting the sliding member (440).

26. The biochemical test strip tube according to any one of claim 19 to claim 25, wherein, the bottom card (410) is made of transparent material; and/or
the first sealing member (420) is a first sealing film, and the first sealing film comprises an aluminum foil film; and/or
the second sealing member (430) is a second sealing film, and the second sealing film comprises an aluminum foil film.
